# FASCICULE DE BREVET EUROPEEN

(11) **EP 3 994 720 B1**
(45) Date de publication et mention de la délivrance du brevet: **02.08.2023**
(21) Numéro de dépôt: 20739284.6
(22) Date de dépôt: 30.06.2020
(51) Int. Cl.: H01L 21/027, H01L 29/66, H01L 29/786

(54) **PROCEDE DE FABRICATION PAR LITHOGRAPHIE**
LITHOGRAFISCHES HERSTELLUNGSVERFAHREN
LITHOGRAPHY PRODUCTION METHOD

(30) Priorité: 02.07.2019 FR 1907342
(43) Date de publication de la demande: 11.05.2022
(73) Titulaire: Centre national de la recherche scientifique, 75016 Paris (FR); Ecole Supérieure de Physique et de Chimie Industrielles de la Ville de Paris, 75005 Paris (FR); SORBONNE UNIVERSITE, 75006 Paris (FR)
(72) Inventeur: BOCKELMANN, Ulrich, 75013 Paris (FR); MENSAH, Kokoura, 91300 Massy (FR)
(74) Mandataire: IPAZ
(86) Numéro de dépôt international: PCT/EP2020/068391
(87) Numéro de publication internationale: WO 2021/001363

(56) Documents cités:
- JP-A- H0 627 635
- US-A1- 2016 013 294
- US-A1- 2018 315 750

## Description

### Domaine technique

La présente invention concerne un procédé de fabrication par lithographie. Elle concerne aussi un dispositif fabriqué par un tel procédé, et un procédé d'utilisation d'un tel dispositif.

Un tel procédé de fabrication permet à un utilisateur de fabriquer un dispositif comprenant une couche conductrice. Le domaine de l'invention est plus particulièrement mais de manière non limitative celui des transistors ou des biocapteurs comprenant un conducteur bidimensionnel tel que du graphène.

### Etat de la technique antérieure

L'année 2004 a témoigné la naissance d'un nouvel allotrope du carbone: le graphène. En effet, c'est dans cette année que l'équipe de Geim et Novoselov a réussi à isoler pour la première fois une seule couche de carbone graphitique par exfoliation mécanique du graphite. La synthèse de ce matériau a constitué un événement majeur puisque depuis 1966 (théorème de Mermin-Wagner), on croyait que l'existence d'un cristal bidimensionnel à température non nulle était impossible.

Le graphène possède des propriétés physiques extraordinaires comme une mobilité très élevée des porteurs de charge (µ > 100000 cm².V⁻¹.s⁻¹ dans le cas du graphène suspendu), une conductivité thermique élevée (estimée à 50 W.cm⁻¹.K⁻¹), une très forte résistance mécanique (constante de raideur k= 50 eV.A⁻² et module de Young d'environ 1TPa). Par ailleurs, le graphène est optiquement transparent et n'absorbe que 2% de la lumière blanche.

Pour des applications en électronique et en détection de biomolécules, il est important de préparer une couche homogène d'une surface suffisante pour structurer celle-ci en dispositifs. Parmi les méthodes de fabrication du graphène, la croissance en phase vapeur CVD (Chemical Vapor Déposition) permet d'avoir une large surface de graphène monocouche. L'exfoliation mécanique est couramment utilisée dans la recherche ; cette approche permet aussi d'avoir un graphène de bonne qualité, mais est limitée à de très petits morceaux. Quelle que soit la technique de préparation, le graphène reste très sensible à son environnement immédiat.

Pour fabriquer des transistors et biocapteurs performants il est important que le graphène soit non dopé, i.e. présente un point de Dirac proche de zéro volt. Le dopage du graphène est typiquement causé par l'introduction des produits organiques ou l'adsorption d'autres impuretés dans le graphène lors du procédé de fabrication du biocapteur et/ou lors de l'utilisation du biocapteur alors qu'il est exposé à un électrolyte. La mobilité des porteurs de charges du graphène est réduite par la diffusion de ces défauts. La sensibilité d'une détection de biomolécule par effet de champs est maximale lorsque le graphène est intrinsèque (non-dopé). Dans cette détection le contrôle du point de travail du dispositif se fait à travers un électrolyte. Ceci induit des limitations de la gamme de tension de mesure. En effet, au-delà de [-1V,1V], on observe des effets électrochimiques à l'interface entre la couche de passivation et l'électrolyte. La dégradation de cette couche conduit à des mesures non reproductibles et des dérives non contrôlées. Par conséquent, il est important que le point de Dirac du dispositif soit situé proche d'un voltage zéro.

Le document US 2018 315750 décrit un dispositif de mesure utilisant une couche de graphène. Le document JP H06 27635 décrit un procédé de masquage utilisant deux couches de résine.

Le but de la présente invention est de proposer, par rapport à l'état de l'art, un procédé de fabrication d'un dispositif comprenant une couche conductrice plus performant.

Un autre but est de fournir un dispositif comprenant une couche conductrice plus performant.

Un autre but est de fournir un procédé d'utilisation d'un tel dispositif.

### Exposé de l'invention

Cet objectif est atteint avec un procédé de fabrication d'un dispositif comprenant :
- une fourniture d'une couche conductrice entre un substrat et une couche de protection,
- un dépôt d'une première couche de résine de lithographie au-dessus ou sur la couche de protection de sorte que la couche de protection soit comprise entre la couche conductrice et la première couche de résine,
- une première lithographie comprenant :
   a) un retrait, dans au moins une zone de retrait de la première lithographie, de la superposition de la première couche de résine, de la couche de protection et de la couche conductrice, et
   b) une conservation, dans au moins une zone de conservation de la première lithographie, de la superposition de la première couche de résine, de la couche de protection et de la couche conductrice, et
- un dépôt, au moins sur l'au moins une zone de conservation de la première lithographie, de préférence sur l'au moins une zone de retrait et sur l'au moins une zone de conservation de la première lithographie, d'une deuxième couche de résine de lithographie sans retirer la première couche de résine de l'au moins une zone de conservation de la première lithographie, et
- une deuxième lithographie comprenant :
   a) un retrait, dans au moins une zone de retrait de la deuxième lithographie, de la superposition de la deuxième résine, de la première résine, de la couche de protection mais pas de la couche conductrice, et
   b) une conservation, dans au moins une zone de conservation de la deuxième lithographie, de la superposition de la deuxième résine, de la première résine, de la couche de protection et de la couche conductrice.

De préférence, la couche conductrice comprend ou consiste en un conducteur bidimensionnel ayant une mobilité supérieure ou égale à 1000 cm².V⁻¹s⁻¹ , consistant de préférence en une couche de graphène, de silicène, de disulfite de molybdène (MoS₂), de germanène, de phosphorène, de stannène, ou de borophène .

De préférence, la première lithographie est une lithographie respectivement positive ou négative et la deuxième lithographie est une lithographie respectivement négative ou positive, la première couche de résine et la deuxième couche de résine étant constituées d'une seule et même résine compatible pour une lithographie positive et pour une lithographie négative.

De préférence, la résine de la première couche de résine et la résine de la deuxième couche de résine sont agencées pour réagir, lors d'une étape de lithographie, au même développeur et/ ou à la même durée, puissance et longueur d'onde d'un rayonnement de préférence ultra-violet.

De préférence, le substrat comprend ou consiste en du silicium et/ou de l'oxyde de silicium.

De préférence, la couche de protection comprend ou consiste en de l'aluminium oxydé, de préférence de l'alumine Al₂O₃.

De préférence, le procédé de fabrication selon l'invention comprend en outre, dans l'au moins une zone de retrait de la deuxième lithographie, un dépôt d'au moins une électrode en contact électrique avec la couche conductrice.

De préférence, le procédé de fabrication selon l'invention comprend en outre, après le dépôt de l'au moins une électrode, un retrait, dans l'au moins une zone de conservation de la deuxième lithographie, de la superposition de la deuxième résine, de la première résine, (optionnellement de la couche de protection) mais pas de la couche conductrice. De préférence, le procédé de fabrication selon l'invention comprend en outre, après ce retrait, un dépôt d'une couche de passivation en contact avec la couche conductrice de l'au moins une zone de conservation de la deuxième lithographie et en contact avec au moins une partie de chaque électrode.

De préférence, la couche de passivation comprend une superposition de deux matières différentes.

De préférence, la couche de passivation comprend une superposition :
- de Al₂O₃ en contact avec la couche conductrice, puis
- d'oxyde de silicium en contact avec l' Al₂O₃ de la couche de passivation, de sorte que l' Al₂O₃ de la couche de passivation soit compris entre la couche conductrice et l'oxyde de silicium de la couche de passivation.

De préférence, la couche de passivation à un champ de claquage supérieur ou égal à 10⁶ V.m⁻¹.

Suivant encore un autre aspect de l'invention, il est proposé un dispositif, de préférence obtenu par un procédé de fabrication selon l'invention, et/ou comprenant :
- un substrat,
- une couche conductrice répartie en au moins une zone de mesure,
- au moins deux électrodes distinctes en contact avec la couche conductrice caractérisé en ce que :
- la couche conductrice consiste en une couche d'un conducteur bidimensionnel ayant un niveau de dopage inférieur à 10¹¹ impuretés (de préférence des impuretés chargées) par cm², et/ou un décalage du point de Dirac par rapport à 0 volt inférieur à 1 Volt en valeur absolue,
- il comprend en outre une couche de passivation ayant un champ de claquage supérieur ou égal à 10⁶ V.m⁻¹ et recouvrant la couche conductrice et au moins une partie de chaque électrode, de sorte que la couche conductrice et au moins une partie de chaque électrode se trouve entre le substrat et la couche de passivation.

De préférence, la couche conductrice est répartie en plusieurs zones de mesure distinctes.

De préférence, les électrodes comprennent une source commune en contact avec toutes les zones de mesure et un drain par zone de mesure, chaque drain n'étant en contact qu'avec une seule zone de mesure.

De préférence, le dispositif selon l'invention comprend en outre un réservoir agencé pour accueillir un électrolyte au-dessus de la couche conductrice, de préférence de sorte que l'électrolyte soit en contact avec la couche de passivation ou avec la couche conductrice.

De préférence, le dispositif selon l'invention comprend des moyens pour mesurer et/ou imposer une tension et/ou une intensité électrique entre l'électrolyte et une électrode source.

De préférence, le dispositif selon l'invention comprend des moyens pour mesurer et/ou imposer une tension et/ou une intensité électrique entre une grille et une électrode source. La grille peut typiquement comprendre le substrat ou une électrode réalisée sur la zone de mesure ou sur chaque zone de mesure de la couche conductrice.

De préférence, le dispositif selon l'invention comprend des moyens pour mesurer et/ou imposer une tension et/ou une intensité électrique entre une électrode source et une électrode drain.

De préférence, le dispositif selon l'invention comprend une couche de polymère et/ou de nucléotides au-dessus de l'au moins une zone de mesure, en contact avec la couche conductrice ou en contact avec la couche de passivation.

Suivant encore un autre aspect de l'invention, il est proposé un procédé d'utilisation d'un dispositif selon l'invention ou obtenu par un procédé de fabrication selon l'invention, comprenant :
- une étape de détection de fixation d'atomes ou de molécules (ou même de cellules biologiques) sur ou au-dessus de la couche conductrice ou de la couche de passivation, et/ou
- une étape d'une détection d'une activité électrique d'au moins une cellule biologique, comme par exemple d'au moins un neurone, sur ou au-dessus de la couche conductrice ou de la couche de passivation.

De préférence, la fixation est une fixation d'atomes ou de molécules (ou même de cellules biologiques) sur une couche de polymère et/ou de nucléotides fixée sur la couche conductrice ou sur la couche de passivation.

Par nucléotide, on entend de préférence une chaine de nucléotides et/ou un acide nucléique, par exemple de l'ADN (Acide désoxyribonucléique) ou ARN (Acide ribonucléique) ou PNA (Acide nucléique peptidique).

De préférence, la fixation est une hybridation de nucléotides sur une couche de polymère et de nucléotides située au-dessus de l'au moins une zone de mesure, et en contact avec la couche conductrice ou en contact avec la couche de passivation.

### Description des figures et modes de réalisation

D'autres avantages et particularités de l'invention apparaîtront à la lecture de la description détaillée de mises en oeuvre et de modes de réalisation nullement limitatifs, et des dessins annexés suivants :
**[****Fig. 1****]** la figure 1 est une vue de coupe de profil de différentes étapes d'un premier mode de réalisation de procédé de fabrication selon l'invention (qui est le mode de réalisation préféré de procédé de fabrication selon l'invention) pour fabriquer un premier mode de réalisation de dispositif 1 selon l'invention (qui est le mode de réalisation préféré de dispositif selon l'invention),
**[****Fig. 2****]** la figure 2 est une vue de dessus de différentes étapes du premier mode de réalisation de procédé de fabrication selon l'invention pour fabriquer le dispositif 1,

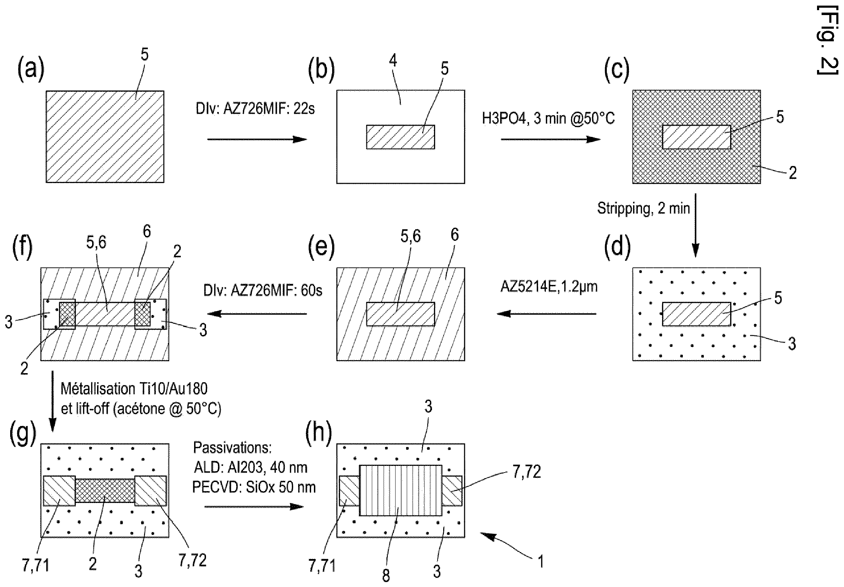

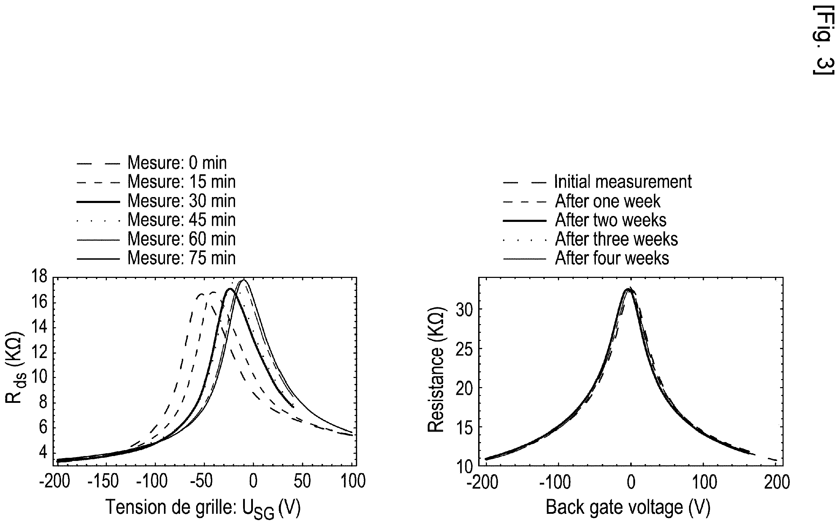

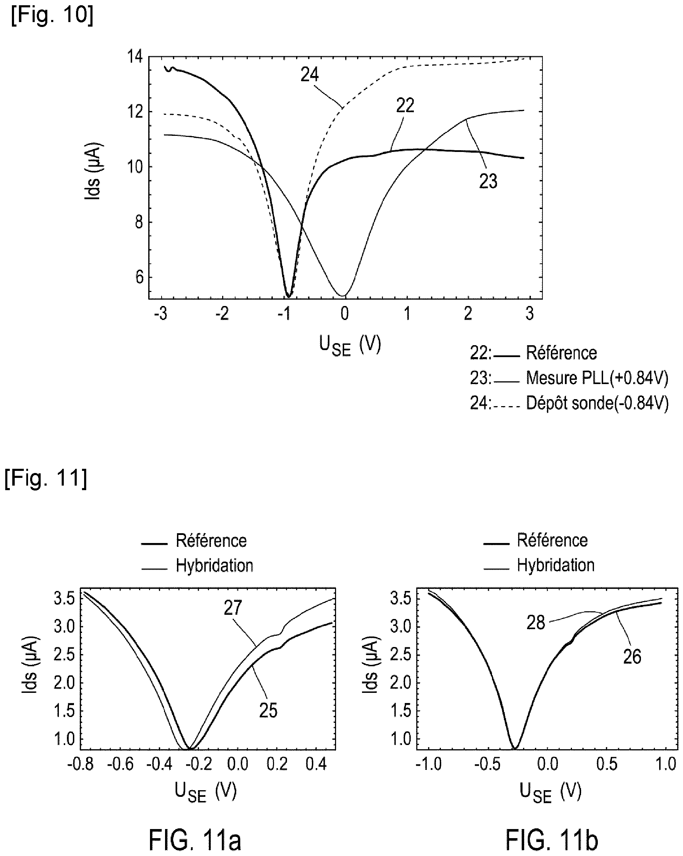

**[****Fig. 3****]** la figure 3 illustre la dérive temporelle de la résistance d'une zone de mesure de la couche conductrice de dimensions, dans le plan de la figure 2, 10µm sur 25 µm (en ordonnée) par rapport à la tension appliquée entre la grille arrière et la source (« Back gate voltage » ; dans cette configuration on fait des mesure de -200V à 200V) (en abscisse) du dispositif 1, pour une couche de passivation 8 ne comprenant que 40 nm de Al₂O₃ (dépôt par ALD) sur sa partie de gauche et pour une couche de passivation 8 comprenant 40 nm de Al₂O₃ (dépôt par ALD) puis 50 nm de Si0₂ (dépôt par PECVD pour « dépôt chimique en phase vapeur assisté par plasma » ou « Plasma-Enhanced Chemical Vapor Déposition » en anglais) pour sa partie de droite,
**[****Fig. 4****]** la figure 4 est une photographie de dessus du dispositif 1 avec un agrandissement 31 de sa zone centrale 32,
**[****Fig. 5****]** la figure 5 illustre la dépendance en tension de grille de la résistance d'une feuille de graphène monocouche 2 dans le dispositif 1 selon l'invention (courbe 15) et dans un autre dispositif en dehors du cadre de la présente invention (courbe 16),
**[****Fig. 6****]** la figure 6 illustre une mesure avant (courbe 29) et après (courbe 36) le recuit d'un transistor individuel à gauche et une mesure avant (courbe 30) et après (courbe 37) le recuit de l'ensemble du réseau de transistors à droite, dans un autre dispositif en dehors du cadre de la présente invention
**[****Fig. 7****]** la figure 7 illustre les données expérimentales (courbe 17) et la formule théorique (courbe 18) de la résistance totale Rtotal du canal d'un transistor aussi appelé zone de mesure du dispositif 1,
**[****Fig. 8****]** la figure 8 illustre le décalage du point de Dirac d'un transistor ou zone de mesure du dispositif 1, à gauche en fonction de la concentration en sel monovalent de l'électrolyte 10 et à droite en fonction du pH de l'électrolyte 10,
**[****Fig. 9****]** la figure 9 illustre la réponse d'un transistor ou zone de mesure du dispositif 1 lors de l'adsorption de la polylysine ; on a Vds = 0,8 V,
**[****Fig. 10****]** la figure 10 illustre la réponse d'un transistor ou zone de mesure du dispositif 1 suite à l'adsorption de la polylysine et la fixation des sondes ; on a Vds = 60 mV.,
**[****Fig. 11****]** la figure 11 illustre la réponse d'un transistor ou zone de mesure du dispositif 1 à l'hybridation d'ADN. A gauche, un transistor sur lequel on a fixé des sondes et à droite un transistor témoin sur lequel on n'a pas fixé les sondes ; Vds = 10 mV
**[****Fig. 12****]** la figure 12 illustre la réponse d'un même transistor ou zone de mesure du dispositif 1 suite à la variation de la concentration des cibles ; à gauche l'hybridation est faite avec une concentration de cibles de 20 nM et à droite, la concentration est de 40 nM; Vds = 10 mV
**[****Fig. 13****]** la figure 13 illustre la réponse d'un transistor ou zone de mesure du dispositif 1 ; l'hybridation est faite avec une concentration de cibles de 1 nM; Vds = 50 mV
**[****Fig. 14****]** la figure 14 illustre la réponse d'un transistor ou zone de mesure du dispositif 1 lors d'une hybridation à bas sel (KCL 10 mM). La concentration des cibles est de 20 nM; Vds = 10 mV
**[****Fig. 15****]** la figure 15 illustre la réponse de deux transistors du dispositif 1 lors des hybridations sens et anti-sens ; à gauche la détection de ARS3 et à droite la détection de ARS5; Vds = 90 mV
**[****Fig. 16****]** la figure 16 illustre le comportement d'un transistor du dispositif 1 en temps réel lors de l'hybridation ; à gauche, l'évolution de la caractéristique, avec cibles complémentaires et à droite, les décalages du point de Dirac avec cibles complémentaires et sans cibles; Vds = 50 mV
**[****Fig. 17****]** la figure 17 illustre la réponse d'un transistor ou zone de mesure du dispositif 1 en temps réel lors de l'hybridation ; à droite, l'évolution de la caractéristique avec cibles complémentaires, et à gauche, les décalages du point de Dirac avec cibles complémentaires et avec cibles non complémentaires; Vds = 50 mV et
[Fig. 18] la figure 18 illustre la mesure avant (référence) et après hybridation pour une détection de produits THRCA, et
[Fig. 19] la figure 19 illustre la réponse d'un transistor ou zone de mesure du dispositif 1 à l'hybridation d'ADN, à très faible concentration (10fM).

Ces modes de réalisation n'étant nullement limitatifs, on pourra notamment considérer des variantes de l'invention ne comprenant qu'une sélection de caractéristiques décrites ou illustrées par la suite isolées des autres caractéristiques décrites ou illustrées (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique de préférence fonctionnelle sans détails structurels, et/ou avec seulement une partie des détails structurels si cette partie uniquement est suffisante pour conférer un avantage technique ou à différencier l'invention par rapport à l'état de la technique antérieure.

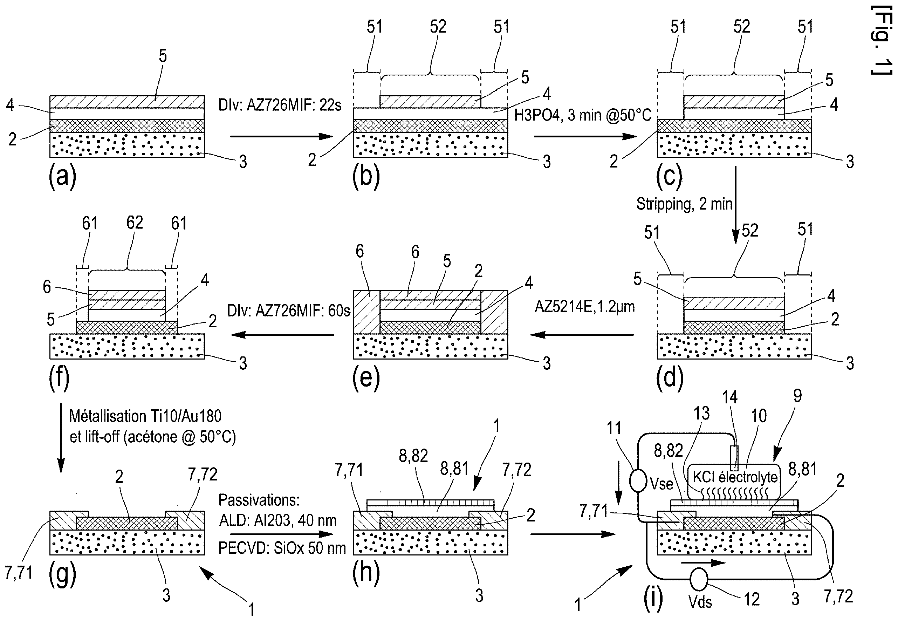

On va tout d'abord décrire, en référence aux figures 1 à 18, un premier mode de réalisation de procédé de fabrication selon l'invention, un premier mode de réalisation de dispositif 1 selon l'invention obtenu par ce procédé, et un premier mode de réalisation de procédé selon l'invention d'utilisation du dispositif 1.

Toutes les étapes suivantes de fabrication ont été soigneusement réalisées en salle blanche.

Dans la suite de la description, l'abréviation rpm signifie "rotations per minute" ou en français « tour(s) par minute ».

### Fourniture de la couche conductrice sous sa couche de protection

Ce mode de réalisation de procédé de fabrication d'un dispositif 1 comprend une fourniture d'une couche conductrice 2 (d'épaisseur typiquement égale à la taille d'un atome de carbone qui est de l'ordre de 0,34nm, de préférence supérieure à 0,1 nm et/ou inférieure à 10 nm ou même 1 nm) entre un substrat 3 et une couche de protection 4 (d'épaisseur typiquement supérieure à 0,5 nm ou même 1 nm et/ou inférieure à 10 nm ou même 5 nm).

Le substrat 3 comprend ou consiste en du silicium et/ou de l'oxyde de silicium, de préférence du silicium (de préférence dopé, de préférence le type de dopant est N/As, orientation : 100±0,5°, Résistivité : 0,005Ω.cm, épaisseur : 525±20µm, Diamètre 100±0,3mm) avec une couche de silicium oxydé (d'épaisseur typiquement supérieure à 100 nm et/ou inférieure à 5 µm notamment dans le cas d'utilisation de la « tension de grille » ou « Back gate voltage ») en contact de la couche conductrice 2.

La couche de protection 4 comprend ou consiste en de l'aluminium oxydé, de préférence de formule Al₂O_{y} avec y un entier 1≤y≤3, de préférence de l'alumine Al₂O₃.

Au stade de la fourniture de la couche conductrice 2 entre le substrat 3 et la couche de protection 4, le substrat 3 est en contact de la couche conductrice 2 (mais pas d'autres couches, en particulier pas de la couche de protection 4).

La couche conductrice 2 est en contact du substrat 3 et de la couche de protection 4.

Cette étape de fourniture se déroule de la manière suivante.

La couche conductrice 2 comprend ou consiste en un conducteur bidimensionnel ayant une mobilité (i.e. mobilité des porteurs de charge dans le plan du conducteur bidimensionnel) supérieure ou égale à 1000 cm².V⁻¹s⁻¹ , consistant de préférence en une couche de graphène, de silicène, de disulfite de molybdène (MoS₂), de germanène, de phosphorène, de stannène, ou de borophène.

Dans un but illustratif mais non limitatif, le choix du graphène est fait pour la couche 2 dans le présent mode de réalisation. Pour toute la suite de la description, chacun des termes couche conductrice 2, conducteur bidimensionnel ou graphène pourra être remplacé par couche conductrice 2, conducteur bidimensionnel, graphène, silicène, disulfite de molybdène (MoS2), germanène, phosphorène, stannène, ou borophène tout en maintenant valable la description de la présente invention.

Nous avons utilisé le graphène CVD (pour « dépôt chimique en phase vapeur » ou « Chemical Vapor Déposition ») pour la couche 2. Le graphène CVD est fabriqué par la déshydrogénation catalytique du méthane à haute température. Les atomes de carbone peuvent alors se précipiter sur une feuille de cuivre et former une couche d'épaisseur monoatomique dépendant des conditions de la croissance. Pour utiliser ce graphène, on a besoin de le transférer sur un substrat 3 : une couche 3 de SiOz par exemple. Le transfert du graphène 2 est une étape essentielle lors de la fabrication du réseau de transistors à effet de champs à base du graphène. Pour avoir un réseau dont tous les transistors marchent ou du moins la majorité, il faut que la feuille de graphène 2 soit continue; ceci passe par un transfert de qualité.

L'eau des robinets de la salle blanche utilisée contient des gaz dissouts (O₂, CO₂....). Son utilisation pour rincer lors du transfert du graphène 2 conduit à la formation des agrégats de bulles de gaz à l'interface graphène-eau. Ces bulles de gaz lors du séchage éclatent et laissent une ouverture dans la feuille du graphène 2. Pour éviter cela, nous puisons l'eau dans un grand bécher qu'on met de côté pendant une journée avant de l'utiliser. Ceci permet de dégazer l'eau c'est-à-dire que les bulles de gaz montent et éclatent à la surface.

Lors de la croissance, le graphène se forme sur les deux faces de la feuille de cuivre mais le graphène obtenu sur chacune des deux faces de ladite feuille de cuivre n'est pas de même qualité ; celui de la face supérieure est de meilleure qualité. En effet, la face inférieure étant posée contre le four, elle ne subit pas les mêmes traitements thermiques. Malgré les propriétés mécaniques exceptionnelles du graphène, son épaisseur de quelques couches atomiques rend difficile, voir impossible, le transfert d'échantillons de graphène de l'ordre du centimètre sans entraîner la rupture du matériau. Pour cette raison la plupart des transferts de graphène sont réalisés en ajoutant un support mécanique pour faciliter sa manipulation. Le matériau le plus utilisé pour transférer du graphène obtenu par croissance sur du cuivre est une couche de polymère, de préférence le polyméthacrylate de méthyle (PMMA). Ce polymère peut être appliqué par simple dépôt par tournette (spin coating) à une vitesse de rotation variant entre 1000 et 4000 tours par minute (rpm). L'épaisseur de la couche du polymère peut être également ajustée en utilisant de la PMMA de masse moléculaire variée, typiquement entre 450 000 et 996 000 g/mol et en utilisant différentes concentrations de la PMMA. L'optimisation de ces paramètres permet d'obtenir une couche de PMMA d'épaisseur idéale, suffisamment résistante mécaniquement tout en demeurant flexible, ce qui garantit une bonne conformité entre le graphène 2 et le substrat de cuivre. Après le dépôt, la couche de PMMA est recuite pendant 6 minutes à une température d'environ 160°C.

Comme mentionné plus haut, les deux faces de la feuille de cuivre sont exposées aux précurseurs et il y a donc croissance sur chacune. Afin d'éviter que la deuxième couche « inférieure » de graphène vienne adhérer à la première couche « supérieure » de graphène lors du transfert, il est préférable de la retirer avant de dissoudre le support métallique. Le graphène de la face inférieure peut être alors gravé par RIE (pour « gravure ionique réactive » ou Reative-Ion Etching) à une pression de 10 nbar pendant 2 minutes.

Pour ce qui concerne la dissolution du support métallique de cuivre, différentes solutions chimiques peuvent être utilisées. Bien qu'il soit possible d'utiliser des acides concentrés tel que l'acide nitrique, leur utilisation n'est pas recommandée, car il y a généralement production de bulles d'hydrogène lors de la dissolution du métal qui risquent d'endommager le graphène. Pour la dissolution du nickel, il est possible d'utiliser une solution aqueuse de chlorure de fer (III) (FeCl₃) à une concentration de 1 M ou alors une solution de HCl diluée à 3 %. Pour ce qui est du cuivre, plusieurs solutions peuvent être utilisées, telles qu'une solution de nitrate de fer(III) (Fe(NO₃)₃) à une concentration de 0,05 g/ml, une solution commerciale pour la gravure du cuivre (CE-100, de la compagnie Transene) ou alors de préférence une solution de persulfate d'ammonium à 0,05-0,1 M.

Lorsque la feuille de cuivre est complètement dissoute, le film de l'ensemble PMMA-graphène flottant à la surface de la solution est ensuite rincé plusieurs fois dans l'eau désionisée préalablement dégazée puis récupéré directement sur le substrat désiré 3, ici du Si/SiO₂ traité comme décrit au paragraphe suivant. Les échantillons sont ensuite séchés sous hotte pendant quelques heures. Pour améliorer l'adhésion du graphène 2 au substrat 3 il est conseillé de chauffer de façon douce l'échantillon jusqu'à 160°C. On peut maintenant dissoudre la PMMA dans l'acétone à 50°C pendant minimum 15 minutes puis rincer à l'isopropanol (IPA). L'utilisation de la soufflette est prohibée une fois que le graphène 2 est transféré pour éviter de l'endommager. Avant toute lithographie, le graphène 2 doit être protégé pour éviter son contact direct avec la résine et le développeur. Pour cela, nous utilisons une couche 4 de 1nm d'oxyde d'aluminium, déposée par la technique de ALD (Dépôt de couche atomique ou « Atomic Layer Déposition »). Le dépôt par ALD est un dépôt séquentiel d'atomes d'aluminium provenant du précurseur le triméthylaluminium (TMA) et d'atomes d'oxygène provenant de l'eau. Pour un dépôt de 1nm, il faut 10 cycles (à raison de 1A°/cycle) à une pression de 300mTorr et à une température de 175°C. Nous expliquerons plus tard comment procéder pour que cette couche 4 protège efficacement le graphène.

Le nettoyage du substrat 3 permet d'avoir une bonne qualité de surface. L'élimination des déchets organiques se fait par le nettoyage à base des solvants standards. Un premier bain d'acétone pendant 5 minutes permet d'enlever ces particules organiques, notamment les résines. Ensuite dans l'isopropanol ; ce dernier bain permet de retirer l'acétone et d'enlever toutes les traces lors du séchage. Une dernière étape de plasma oxygène est nécessaire afin de rendre hydrophile la surface du substrat. Ceci permet un bon étalement de la feuille de graphène et améliore son adhésion au substrat.

Une fois le transfert réussi, on peut passer maintenant aux étapes de lithographie qui nous permettent de découper le graphène 2 et définir les motifs des électrodes, puis à l'étape de la métallisation.

Dans une alternative, l'assemblage de la couche conductrice 2 entre un substrat 3 et une couche de protection 4 peut être acheté directement assemblé.

### Lithographies

La lithographie est l'étape technologique nécessaire pour transférer les motifs présents sur un masque. Le transfert se fait sur de la résine photosensible étalée sur la surface où on veut imprimer les motifs. Par insolation, la résine exposée réagit et sa structure change. Il est alors possible d'enlever sélectivement soit les parties exposées, soit les parties protégées. Il existe plusieurs types de résine. Les résines positives (dont les parties exposées aux rayons ultraviolets UV seront enlevées) et des résines négatives (dont les parties exposées restent après développement). D'autres résines permettant les deux types de lithographie sont dites réversibles. Elles peuvent se comporter comme une résine positive ou négative selon les traitements d'insolation et de recuit qu'elles subissent. Celle que nous utilisons est la AZ5214E de chez Sigma, dont le constituant principal est le solvant methoxy-propyl acetate (PGMEA), avec une teneur en eau de 0,5%, une viscosité est de 24SI, un spectre de sensibilité est de 310 à 420nm et un coefficient d'absorption à 377nm est de 0,76. Le procédé d'inversion nécessite une étape supplémentaire de recuit et d'insolation pleine plaque.

### Lithographie de préparation du substrat 3

Cette lithographie est réalisée sur le substrat 3 avant le transfert de la couche 2 sur le substrat 3.

Nous utilisons pour ce mode de réalisation un substrat 3 d'oxyde de silicium dont l'oxyde de silicium fait 1 µm d'épaisseur sur une couche épaisse de silicium non oxydé. Bien qu'avec cette épaisseur le contraste optique ne nous permet pas de bien voir le graphène 2, elle nous permet du moins d'empêcher les fuites de courant entre la source et la grille, même pour la taille relativement grande de nos électrodes. Ces substrats 3 sont tout d'abord découpés en de petits morceaux (1,2 cm X 1,2 cm) puis nettoyés à l'acétone pendant 5 minute(s) et rincés à l'IPA. On passe alors à la lithographie qui permet d'imprimer les croix d'alignement sur le substrat 3. Ces croix nous permettront de nous aligner sur les canaux de graphène 2 qui sont totalement invisibles sur 1 µm de SiOz en dessous de la résine. Il faut alors faire une ouverture dans la résine et faire la métallisation. Il s'agit d'une lithographie négative comprenant les étapes suivantes :
a. Recuit du substrat pour évaporer l'eau : 5minutes à 120°C
b. Revêtement résine AZ5214E : 30 s à 4000 rpm
c. Recuit : 2 minutes à 110°C
d. Exposition en utilisant le masque correspondant : Alignement et exposition 2 s. La puissance d'exposition est de l'ordre de □7,5 mW/cm² avec une longueur d'onde λ=420nm
e. Recuit : 2minutes à 125°C
f. Exposition sans masque : exposition « Flood exposure » pendant 15 s. La puissance d'exposition est de l'ordre de □7,5 mW/cm² avec une longueur d'onde λ=420nm
g. Développement : AZ 726 MIF, 35 s. Ce développeur révèle les motifs imprimés sur la résine AZ5214E, et son principal constituant est le « tétraméthyl-ammonium-hydroxyde » TMAH à 2,38%
h. Métallisation : Cr 5 nm/Au 180 nm
i. Décollage (« Lift-off ») : Acétone 50°C, 15minute(s) et rinçage à l'IPA

Les deux lithographies suivantes sont réalisées après le transfert de la couche 2 sur le substrat 3.

### 1^{ère} lithographie des transistors: lithographie de la couche de protection 4 et du graphène 2

Ce mode de réalisation de procédé selon l'invention comprend en outre :
- un dépôt d'une première couche de résine 5 de lithographie (d'épaisseur typiquement supérieure à 1 µm et/ou inférieure à 2 µm, typiquement d'environ 1,2 µm) au-dessus ou sur la couche de protection 4 de sorte que la couche de protection 4 soit comprise entre la couche conductrice 2 et la première couche de résine 5 ; ceci est illustré en partie a) des Figures 1 et 2. La couche de protection 4 est alors en contact de la première couche de résine 5 et de la couche conductrice 2 (mais pas d'autres couches) ; et
- une première lithographie comprenant :
   a) un retrait, dans au moins une zone de retrait 51 de la première lithographie, de la superposition de la première couche de résine 5, de la couche de protection 4 et de la couche conductrice 2, et
   b) une conservation, dans au moins une zone de conservation 52 de la première lithographie, de la superposition de la première couche de résine 5 (qui peut toutefois être partiellement endommagée ou affinée, sans toutefois présence de trou qui donnerait accès à la couche conductrice 2 ou à la couche de protection 4), de la couche de protection 4 et de la couche conductrice 2. Ceci est illustré aux parties b), c), et d) des Figures 1 et 2

L'expression « au-dessus de » la couche A est utilisée pour dire qu'il n'y a pas nécessairement de contact avec la couche A.

L'expression « sur » la couche A est utilisée pour dire qu'il y a contact avec la couche A, c'est-à-dire « en contact de ».

Ainsi, la première lithographie retire de manière sélective des parties de la première résine 5 et conserve de manière sélective d'autres parties de la première résine 5, et retire des parties de la couche de protection 4 superposées aux parties retirées de la première résine 5 et retire des parties de la couche conductrice 2 superposées aux parties retirées de la couche de protection 4.

Cette première lithographie permet de dessiner les futures zones de découpe du graphène 2, typiquement en 48 morceaux de 60 µm sur 10 µm. Il s'agit alors d'imprimer les motifs du masque correspondant en lithographie positive sur le substrat 3 sur lequel le graphène 2 a été déjà transféré et protégé. Ces étapes sont de préférence réalisées de la manière suivante :
a. Revêtement par la couche 5 de résine AZ5214E au-dessus ou de préférence directement sur la couche de protection 4 de sorte que la couche de protection 4 soit comprise entre la couche conductrice 2 et la première couche de résine 5: 30 s à 4000 rpm ; ceci est illustré en partie a) des Figures 1 et 2
b. Recuit : 2 minutes à 110°C
c. Exposition en utilisant le masque correspondant : Alignement et exposition pendant 15 s. La puissance d'exposition est de l'ordre de □7,5 mW/cm² avec une longueur d'onde λ=420nm
d. Développement : AZ 726 MIF, 22 s ; ceci est illustré en partie b) des Figures 1 et 2
e. Gravure de la couche de protection : H₃PO₄ à 50°C, 3 minutes, rinçage à l'eau 2 minutes; ceci est illustré en partie c) des Figures 1 et 2
f. Gravure du graphène non désiré : Décapage, 2 minutes ; ceci est illustré en partie d) des Figures 1 et 2. Pour la gravure du graphène monocouche 2, il faut un plasma oxygène RIE (« Reactive-Ion Etching ») pendant 2 minutes à 10nbar

Contrairement à l'incitation pour l'homme du métier, après cette dernière étape on ne dissout pas la résine 5 sur les 48 morceaux de graphène 2 protégés par la couche 4 (1 nm) d'Al₂O₃ (ALD) avant de passer à la deuxième lithographie suivante décrite ci-après. En effet, l'alumine 4 adhère mal au graphène 2 (dû à sa nature hydrophobique), ce qui fait que, quelles que soient les précautions prises lors de cette opération, l'alumine 4 risquerait de s'enlever à certains endroits et d'exposer le graphène 2 à la résine et aux autres déchets organiques pouvant se trouver dans le solvant.

### 2ème lithographie des transistors : lithographie de la couche de protection 4

Ainsi, selon ce mode de réalisation selon l'invention on enrésine à nouveau le substrat 3 avec de la résine 6 en vue de la 2^{ème} lithographie (dite de contact, en vue de la métallisation du graphène 2) qui est une lithographie négative où la résine 5 conservée sur le graphène 2 est retraitée en lithographie négative.

Ce mode de réalisation de procédé selon l'invention comprend ainsi en outre :
- un dépôt, au moins sur l'au moins une zone de conservation 52 de la première lithographie, de préférence sur l'au moins une zone de retrait 51 et sur l'au moins une zone de conservation 52 de la première lithographie, d'une deuxième couche de résine 6 de lithographie (d'épaisseur typiquement supérieure à 1µm et/ou inférieure à 2 µm, typiquement d'environ 1,2 µm) sans retirer la première couche de résine 5 de l'au moins une zone de conservation 52 de la première lithographie ; ceci est illustré en partie e) des Figures 1 et 2 ; La première couche de résine 5 est alors en contact de la deuxième couche de résine 6 et de la couche de protection 4 (mais pas d'autres couches). La deuxième couche de résine 6 est alors en contact de la première couche de résine 5 et du substrat 3 (mais pas d'autres couches, sauf sur les bordures des couches 2 et 4)
- une deuxième lithographie comprenant : a) un retrait, dans au moins une zone de retrait 61 de la deuxième lithographie, de la superposition de la deuxième résine 6 , de la première résine 5, de la couche de protection 4 mais pas de la couche conductrice 2 ; ceci est illustré en partie f) des Figures 1 et 2
   b) une conservation, dans au moins une zone de conservation 62 de la deuxième lithographie, de la superposition de la deuxième résine 6, de la première résine 5, de la couche de protection 4 et de la couche conductrice 2 ; ceci est illustré en partie f) des Figures 1 et 2

On remarque que la première lithographie est une lithographie respectivement positive ou négative et que la deuxième lithographie est une lithographie respectivement négative ou positive, la première couche de résine 5 et la deuxième couche de résine 6 étant constituées d'une seule et même résine compatible pour une lithographie positive et pour une lithographie négative.

On remarque que la résine de la première couche de résine 5 et la résine de la deuxième couche de résine 6 sont agencées pour réagir, lors d'une étape de lithographie, au même développeur et/ ou à la même durée, puissance et longueur d'onde d'un rayonnement de préférence ultraviolet (i.e. de longueur d'onde supérieure à 10 nm et/ou inférieure à 380 nm).

Ce mode de réalisation de procédé selon l'invention comprend en outre :
- dans l'au moins une zone de retrait 61 de la deuxième lithographie, un dépôt d'au moins une (de préférence plusieurs) électrode(s) 7 en contact électrique avec la couche conductrice 2, de préférence, pour chaque zone de mesure, en contact avec une partie mais pas toute la couche conductrice 2.
- après le dépôt de l'au moins une électrode 7, un retrait, dans l'au moins une zone de conservation 62 de la deuxième lithographie, de la superposition de la deuxième résine 6, de la première résine 5, et de manière optionnelle aussi de la couche de protection 4, mais pas de la couche conductrice 2.

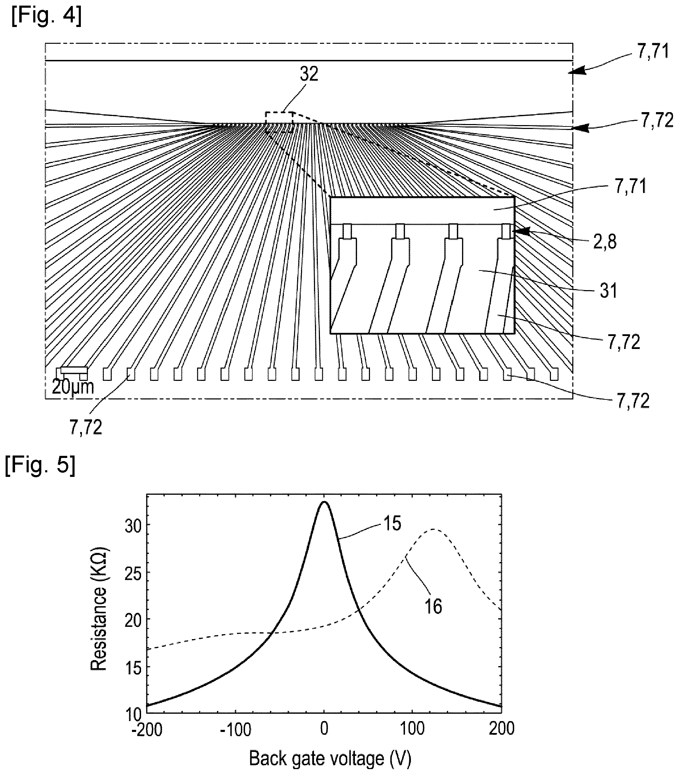

En pratique, comme illustré en Figure 4, on fabrique N (N un entier supérieur à un) zones de mesure i.e. N (quarante-huit dans le présent mode de réalisation) zones de couche conductrice 2 isolées les unes des autres.

On dépose N+1 électrodes 7 :
- une électrode 7, 72 (appelée drain) par zone de mesure en contact de la couche conductrice de cette zone de mesure mais pas de la couche conductrice des autres zones de mesure
- une électrode 7, 71 (appelée source) commune à toute les zones de mesure et en contact de la couche conductrice de chaque zone de mesure

### Ceci est illustré à la partie g) des Figures 1 et 2

Cette deuxième lithographie permet de définir les motifs des électrodes en vue d'une prochaine métallisation. Ces étapes sont de préférence réalisées de la manière suivante :
a. Recuit du substrat pour évaporer l'eau : 5 minutes à 120°C
b. Revêtement résine AZ5214E : 30 s à 4000 rpm
c. Recuit : 2 minutes à 110°C
d. Exposition en utilisant le masque correspondant : Alignement et exposition pendant 2 s. La puissance d'exposition est de l'ordre de □7,5 mW/cm² avec une longueur d'onde λ=420nm
e. Recuit : 2 minutes à 125°C
f. Exposition sans masque : exposition « pleine plaque » (ou « Flood exposure ») pendant 15 s. La puissance d'exposition est de l'ordre de □7,5 mW/cm² avec une longueur d'onde λ=420nm
g. Développement : AZ 726 MIF, 1 minute(s)
h. Gravure de la couche de protection sur les contacts : H₃PO₄ à 50°C, 3 minute(s), rinçage à l'eau 2 minutes
i. Dépôt métallique (Ti10/Au180) par évaporation
j. Décollage : Acétone 50°C, 15 minutes et rinçage à l'IPA

Chaque électrode comprend donc typiquement une première couche de titane (d'épaisseur typiquement supérieure à 4 nm et/ou inférieure à 20 µm, typiquement de 10 nm) sur laquelle située une deuxième couche d'or (d'épaisseur typiquement supérieure à 50 nm et/ou inférieure à 250 nm, typiquement de 180 nm).

### Passivation

Ce mode de réalisation de procédé selon l'invention comprend en outre, après le retrait (dans l'au moins une zone de conservation 62 de la deuxième lithographie) de la superposition de la deuxième résine 6 et de la première résine 5 (et de la couche de protection 4) mais pas de la couche conductrice 2, un dépôt d'une couche de passivation 8 (d'épaisseur typiquement supérieure à 10 nm et/ou inférieure à 400 nm, typiquement de 90 nm) en contact avec la couche conductrice 2 et/ou de la couche de protection 4 de l'au moins une zone de conservation 62 de la deuxième lithographie et en contact avec au moins une partie de chaque électrode 7.

### Ceci est illustré à la partie h) des Figures 1 et 2

La couche de passivation 8 comprend un oxyde et/ou un nitride (par exemple du Si₃N₄).

La couche de passivation 8 comprend une superposition de deux matières diélectriques différentes.

La couche de passivation 8 (d'épaisseur typiquement supérieure à 10 nm et/ou inférieure à 400 nm, typiquement de 90 nm) comprend de préférence une superposition de deux oxydes différents.

La couche de passivation 8 comprend une superposition :
- d'une première sous couche de passivation 81 (d'épaisseur typiquement supérieure à 5 nm et/ou inférieure à 200 nm, typiquement de 40 nm), comprenant un mélange d'atomes d'oxygène et d'aluminium, de préférence de formule Al₂Oₓ avec x un entier 1≤x≤3, comprenant ou consistant de préférence en de l'Al₂O₃, et en contact avec la couche conductrice 2. Cette couche 81 est déposée par ALD (400cycles, T=175°C, P=300mTorr) ; puis
- d'une deuxième sous couche de passivation 82 (d'épaisseur typiquement supérieure à 5 nm et/ou inférieure à 200 nm, typiquement de 50 nm), comprenant un mélange d'atomes d'oxygène et de silicium, comprenant ou consistant de préférence en de l'oxyde de silicium de préférence de formule SiO_{z} avec z un entier 1≤z≤2, et en contact avec la première sous couche de la couche de passivation, de sorte que la première sous couche 81 de la couche de passivation soit comprise entre la couche conductrice 2 et la deuxième sous couche 82 ; cette couche 82 est déposée par PECVD (T=280°C, Chauffage: 1min, -Stabilisation: 10s, -, -Préparation de surface: 10s, -Temps à dépôt: 5s, -Envoi SiH₄: 5s, -Dépôt: 1min 53 s)

La couche de passivation 8 a un champ de claquage supérieur ou égal à 10⁶ V.m⁻¹ .

Il est en effet préférable de protéger le mode de réalisation de dispositif selon l'invention 1 obtenu contre les impuretés, les molécules d'eau dans l'air ambiant et de stabiliser ses caractéristiques électriques.

Le choix de la nature et de l'épaisseur de l'oxyde de la couche de passivation 8 dépend de sa résistance à l'électrolyte de mesure 10 lors de la détection électronique et de son étanchéité. Nous avons combiné deux types d'oxyde : l'oxyde d'aluminium Al₂O₃ déposé par ALD et l'oxyde de silicium déposé par PECVD.

En effet l'alumine déposée par ALD est de très bonne qualité mais ne stabilise pas les caractéristiques électriques des transistors et est en plus vite détruite par l'électrolyte 10 lors de la détection électronique. Elle est utilisée uniquement pour ses propriétés d'étanchéité. Sa combinaison avec l'oxyde de silicium permet de stabiliser les dispositifs et de faire des mesures de détection en se limitant à [-1V,1V] pour la tension source-électrolyte ; quand on dépasse cette gamme de tension les phénomènes électrochimiques apparaissent.

La figure 3 illustre l'avantage technique d'une telle couche de passivation 8 comprenant une superposition de deux oxydes différents :
- la partie gauche de la figure 3 illustre, pour une couche de passivation 8 ne comprenant que 40 nm d'Al₂O₃ (dépôt par ALD), la dérive temporelle de la résistance d'un canal (en ordonnée) par rapport à la tension grille-source (en abscisse) obtenue dans les conditions expérimentales suivantes : température ambiante, sans électrolyte 10.
- la partie droite de la figure 3 illustre, pour une couche de passivation 8 comprenant 40 nm d'Al₂O₃ (dépôt par ALD) puis 50 nm de Si0₂ (dépôt par PECVD), la dérive temporelle de la résistance d'un canal (en ordonnée) par rapport à la tension grille-source (en abscisse) obtenue dans les conditions expérimentales suivantes : température ambiante, sans électrolyte 10.

On observe sur la figure 3 à droite une dérive temporelle bien moins importante que sur la partie gauche de la figure 3.

On note que les figures 1 et 2 ne sont que des vues partielles des différentes couches 2, 3, 4, 5, 6, 7 et 8 pour une seule zone de mesure.

En pratique, comme illustré en Figure 4 (pour laquelle la couche de passivation 8 est déjà déposée sur le graphène 2), on fabrique au moins une zone de mesure, de préférence plusieurs zones de mesure i.e. plusieurs (quarante-huit dans le présent mode de réalisation) zones de couche conductrice 2 protégée par une couche de passivation 8 correspondant aux différents (quarante-huit) drains 7, 72.

Ce mode de réalisation de procédé de fabrication selon l'invention peut en outre comprendre un dépôt d'une couche 13 de polymère et/ou de nucléotides en contact de la couche conductrice 2 ou en contact de la couche de passivation 8. La nature et l'utilisation de cette couche 13 est détaillée par la suite.

### Dispositif 1 selon l'invention

Le mode de réalisation de dispositif selon l'invention 1 obtenu par le mode de réalisation de procédé de fabrication selon l'invention précédemment décrit comprend donc :
- le substrat 3,
- la couche conductrice 2 répartie en l'au moins une zone de mesure, typiquement au moins 10 zones de mesure, typiquement 48 zones de mesure sur la figure 4,
- les au moins deux électrodes 7, 71, 72 distinctes et en contact avec la couche conductrice 2.

La couche conductrice 2 consiste de préférence en une couche de conducteur bidimensionnel (de préférence du graphène) ayant (de préférence pour chaque zone de mesure) :
- un niveau de dopage inférieur à 10¹¹ impuretés (de préférence des impuretés chargées) par cm², et/ou
- un décalage de la tension du point de Dirac par rapport à 0 volt inférieur à 1 Volt en valeur absolue, la tension du point de Dirac étant mesurée sur une courbe de variation de la résistance de la couche conductrice 2 d'une seule zone de mesure considérée en fonction de la « tension de grille » ou « Back gate voltage » (c'est-à-dire la tension entre la couche 3 et l'électrode 71) en l'absence d'électrolyte 10, le point de Dirac étant sur cette courbe le point ayant la tension la plus proche de zéro avec une dérivée seconde nulle et/ou une valeur maximum de résistance .

On peut en effet contrôler le dopage du graphène 2 en appliquant un champ extérieur. En changeant le signe de la différence de potentiel, on peut passer continûment d'un dopage en électrons à un dopage en trous. Ainsi pour une tension de grille positive la mobilité est assurée par les électrons et les trous assument la conductivité pour une tension de grille négative. Le point de neutralité entre la conductivité en trous et en électrons (Charge neutrality point) est appelé Point de Dirac.

Chaque « impureté » considérée est :
- chaque présence d'un atome en dehors du réseau cristallographique du conducteur bidimensionnel 2 (ici du graphène), ou
- chaque absence d'atome dans le réseau cristallographique du conducteur bidimensionnel 2 (ici du graphène).

Une impureté est chargée si elle est chargée électriquement, négativement ou positivement.

Le dispositif 1 comprend en outre de préférence la couche de passivation 8 ayant un champ de claquage supérieur ou égal à 10⁶ V.m⁻¹ et recouvrant la couche conductrice 2 et au moins une partie de chaque électrode 7, de sorte que la couche conductrice 2 et au moins une partie de chaque électrode 7 se trouve entre le substrat 3 et la couche de passivation 8. Dans certaines variantes toutefois cette couche 8 peut être absente.

La couche conductrice 2 comprend au moins une zone de mesure, et est de préférence répartie en plusieurs zones de mesure distinctes et séparées les unes des autres.

Chaque zone de mesure consiste en une zone de couche conductrice 2 intercalée entre le substrat 3 et la couche de passivation 8.

Les électrodes 7 comprennent :
- la source commune 7, 71 en contact avec toutes les zones de mesure et
- un drain 7,72 par zone de mesure, chaque drain 72 n'étant en contact qu'avec une seule zone de mesure.

Le dispositif 1 comprend une électrode 14. Cette électrode est typiquement une électrode de Ag/AgCl.

Le dispositif 1 comprend en outre un réservoir 9 agencé pour accueillir un électrolyte 10 (de sorte que l'électrode 14 soit plongée dans cet électrolyte 10) au-dessus de la couche conductrice 2, de préférence de sorte que l'électrolyte 10 soit en contact avec la couche de passivation 8 ou avec la couche conductrice 2 si cette couche de passivation 8 est absente.

Le dispositif 1 comprend des moyens 11 pour mesurer et/ou imposer :
- une tension Use aussi appelée Vse et/ou une intensité Ise électrique entre l'électrolyte 10 (ou l'électrode 14) et l'électrode source 71, et/ou
- une tension U_{SG} aussi appelée V_{SG} et/ou une intensité I_{SG} électrique entre la grille (i.e. le substrat 3, plus exactement la couche dopée et non oxydée du substrat 3 séparée de la couche 2 par la couche oxydée et non dopée du substrat 3) et l'électrode source 71
- une tension U_{SG} aussi appelée V_{SG} et/ou une intensité I_{SG} électrique entre une autre grille possible et l'électrode source 71. Cette autre grille peut être une électrode de grille réalisée sur la zone de mesure ou sur chaque zone de mesure de la couche conductrice 2, de sorte que la couche 2 soit située entre le substrat 3 et cette autre grille. Cette autre grille peut être présente (en l'absence ou en présence de la couche 8 et/ou en l'absence ou en présence de la couche 4) dans une variante de l'invention pour laquelle le procédé de fabrication selon l'invention comprend une fabrication d'une électrode de grille réalisée sur la zone de mesure ou sur chaque zone de mesure de la couche conductrice 2, de sorte que la couche 2 soit située entre le substrat 3 et cette autre grille (avec ou sans fabrication ou présence de la couche 8 et/ou avec ou sans fabrication ou présence de la couche 4).

Le dispositif 1 comprend des moyens 12 pour mesurer et/ou imposer une tension Uds aussi appelée Vds et/ou une intensité électrique Ids entre l'électrode source 71 et une des électrodes drain 72, de préférence à tour de rôle pour chaque électrode drain 72.

Ces moyens 11 et 12 12 comprennent typiquement des moyens électroniques et/ou informatiques, tels que par exemple décrits dans l'article « Spatially resolved electronic détection of biopolymers » Pouthas et al., PHYSICAL REVIEW E70, 031906(2004) (en particulier dans sa figure 2).

Les deux modes, courant imposé et tension imposée, sont possibles. Typiquement, on impose Vds et on mesure Ids, et on impose en outre :
- V_{SG} dans une configuration de grille, la grille étant le substrat 3 ou de manière plus classique l'autre électrode de grille telle que décrite précédemment
- Vse dans une autre configuration.

Le dispositif 1 comprend une couche 13 de polymère et/ou de nucléotides au-dessus de l'au moins une zone de mesure, en contact avec la couche conductrice 2, en contact avec la couche de protection 4 si cette couche est présente, ou en contact avec la couche de passivation 8 si cette couche de passivation est présente.

Le substrat 3 est en contact de la couche conductrice 2 et des électrodes 7, 71, 72 (mais pas d'autres couches, en particulier pas de la couche de passivation 8).

La couche conductrice 2 est en contact du substrat 3 et de la couche de passivation 8 (et d'au moins deux électrodes 7, de préférence de la source 71 et d'un des drains 72).

Le dispositif 1 a été caractérisé, en particulier pour chaque transistor ou zone de mesure, son point de Dirac et sa mobilité comme détaillé ci-dessous.

### Point de Dirac

Le graphène 2 est transféré sur un substrat 3 de P⁺⁺Si/SiO₂ (silicium dopé positivement et recouvert d'une couche d'oxyde de silicium) qui permet de réaliser un effet de champ électrique. En réalité sous la feuille du graphène 2 se trouve 1µm d'oxyde de silicium qui est un isolant, avant d'accéder au silicium non oxydé proprement dit. Celui-ci est très fortement dopé (chimiquement) pour le rendre métallique. Si on applique une différence de potentiel entre le silicium dopé (la grille arrière) et la feuille de graphène (la source), on réalise un condensateur (et en présence des contacts de source et drain un transistor à effet de champs) dont le diélectrique est le dioxyde de silicium. Par effet de champ électrique, en réglant la différence de potentiel, on peut ajouter ou enlever les électrons au graphène 2. Comme le graphène 2 non dopé ne comporte pas de porteurs de charge, on dispose alors d'un « bouton » extérieur pour contrôler électriquement le dopage en porteurs de charge. En changeant le signe de la différence de potentiel, on peut passer continûment d'un dopage en électrons à un dopage en trous. Ainsi pour une tension de grille positive, la mobilité est assurée par les électrons et les trous assument la conductivité pour une tension de grille négative.

La figure 5 illustre la dépendance en « tension de grille » ou « Back gate voltage » (c'est-à-dire entre la couche 3 et l'électrode 71) de la résistance d'une feuille de graphène 2 monocouche (i.e. une seule zone de mesure) :
- pour le dispositif 1 selon l'invention, sur la courbe 15
- pour un dispositif pour lequel la couche de résine 5 est entièrement dissoute à la fin de la première lithographie de la couche de protection 4 et du graphène 2 avant le dépôt de la résine 6, sur la courbe 16.

Nous avons imposé dans ce cas un voltage U_{ds} de 0,5V. La couche 8 est bien présente.

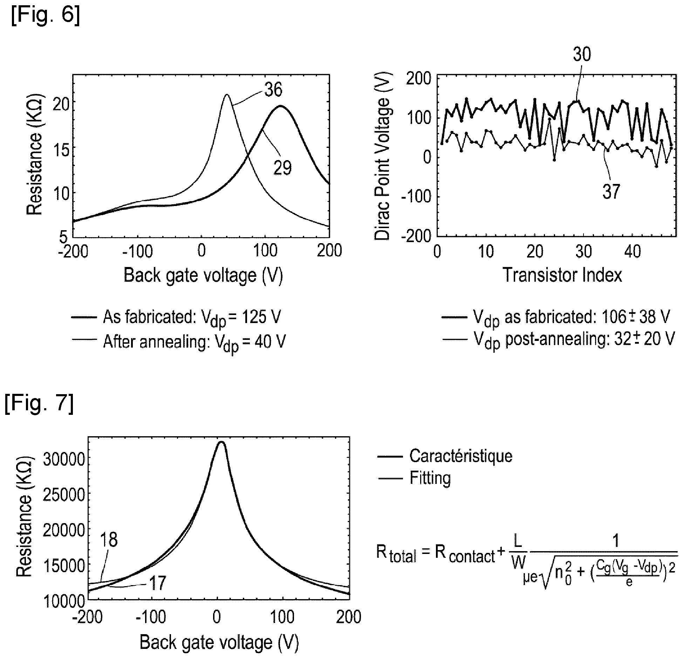

La figure 6 illustre :
- à gauche, la dépendance en « tension de grille » ou « Back gate voltage » (c'est-à-dire entre la couche 3 et l'électrode 71) de la résistance d'une feuille de graphène 2 monocouche (i.e. une seule zone de mesure) pour un autre dispositif pour lequel la couche de résine 5 est entièrement dissoute à la fin de la première lithographie de la couche de protection 4 et du graphène 2 avant le dépôt de la résine 6,
- à droite, la tension du point de Dirac en fonction du numéro (1 à 48) de la zone de mesure (i.e. indice de la zone de mesure aussi appelé indice du transistor) pour cet autre dispositif
avant un recuit, sur les courbes 29 et 30, et après un recuit sur les courbes 36 et 37. Pour un tel recuit, cet autre dispositif est introduit dans le bâti d'un four pendant une journée pour faire un vide autour de 5. 10⁻⁶ mbar. On augmente alors la tension d'alimentation d'une LED qui permet de chauffer par effet Joule l'enceinte où est placé cet autre dispositif. On peut alors monter en température jusqu'à 280°C pour une tension d'alimentation de 36V. Le recuit commence une fois que la température souhaitée est atteinte. Sur le réseau présenté, nous avons fait un recuit de 48 heures à 280°C. On remarque après le recuit une nette diminution du niveau de dopage et une amélioration de la mobilité des transistors.

On remarque la valeur très faible du point de Dirac pour le dispositif 1 selon l'invention, bien plus proche de zéro que la courbe 16 de la Figure 5 (sans recuit) ou que la courbe 36 de la Figure 6 (avec recuit).

L'invention permet une plus basse valeur de tension du point de Dirac (ou « Charge neutrality point » (V_{CNP}) en anglais), et permet d'éviter un recuit qui a pour inconvénient de généralement augmenter la résistance de contact des électrodes 7.

Sur la courbe 15 de la figure 5, la valeur de la tension au point de Dirac est de 0 Volts.

### Mobilité

L'invention évite une diminution de la mobilité, car elle protège le graphène 2 d'une introduction de défauts ou impuretés (chargé(e)s ou non chargé(e)s)

L'estimation de la mobilité des transistors du dispositif 1 se fait en minimisant l'écart quadratique moyen entre les données expérimentales (courbe 17 de la figure 7) et la formule théorique (courbe 18 de la figure 7) qui décrit la résistance totale Rₜₒₜₐₗ du canal. La mobilité des porteurs de charge pour ce transistor est estimée à 3732,5 cm2 V⁻¹ s-¹

La formule utilisée est indiquée à droite de la figure 7, avec :
V_{g} est la tension de grille (le « back gate voltage »).
V_{dp}= 0 V : Tension au point de Dirac.
C_{g}= 34,5.10⁻¹⁰ F.cm⁻² : Capacité de l'oxyde de grille
n₀ = 5,11 . 10¹¹ cm⁻² : Densité de charge des impuretés.
L = 25 µm: Longueur du canal
W = 10 µm: Largeur du canal
µ = 1301,42 cm²/Vs : Mobilité des porteurs de charge
e est la charge élémentaire d'un proton (e= 1,602 . 10⁻¹⁹ C)
n = C_{g} (U_{g}-U_{dp})/e: densité de charge induite par la grille.
Pour U_{g}= i.e., n₀ = - C_{g} U_{dp} /e, pour le graphène intrinsèque, n₀ = 0 i.e.,
U_{dp}=0.
Si U_{g}≤U_{dp}, conduction par les trous.
Si U_{g}≥U_{dp}, conduction par les électrons.
R_{contact}= 9000Ω

### Procédé selon l'invention d'utilisation du dispositif 1.

Le premier mode de réalisation du procédé selon l'invention d'utilisation du dispositif 1 comprend une étape de détection de fixation d'atomes ou de molécules (ou même de cellules biologiques) au-dessus de ou directement sur (c'est-à-dire en contact de) :
- la couche conductrice 2 de chaque zone de mesure si la couche de passivation 8 est absente, ou la couche de protection 4 si cette couche est présente, ou
- la couche de passivation 8 si la couche de passivation 8 est présente.

La fixation est de préférence une fixation d'atomes ou de molécules (ou même de cellules biologiques) sur une couche de polymère et/ou de nucléotides préalablement fixée sur la couche conductrice 2 ou sur la couche de passivation 8.

La fixation est de préférence une hybridation de nucléotides sur une couche 13 de polymère (typiquement de la polylysine) et de nucléotides (de préférence d'oligonucléotides comprenant de 10 à 50 bases, typiquement 20 bases) située au-dessus de l'au moins une zone de mesure, et en contact avec la couche conductrice 2 ou en contact avec la couche de passivation 8.

Pour cette étape de détection, l'électrode 14 est plongée dans l'électrolyte 10.

Dans la configuration où les porteurs de charge créés par effet de champ sont fournis par le silicium dopé 3 jouant le rôle de grille métallique, nous avons modulé la tension de grille sur l'intervalle [-200V,200V]. Le dopage du graphène 2 est alors contrôlé par les trous et les électrons venant du silicium 3. Les propriétés physico-chimiques sont dans cette situation assez stables et n'affectent pas le comportement du graphène 2 d'une mesure à une autre si la stabilisation est bien faite. Nous pouvons aussi inverser les choses en utilisant la couche de passivation (40 nm Al₂O₃ déposé par ALD et 50 nm SiO2 déposé par PECVD) comme diélectrique et un électrolyte 10 jouant le rôle de la grille qui fournit les ions pour contrôler le dopage du graphène. Dans ce cas on peut facilement modifier les propriétés de l'électrolyte 10 et voir comment se comporte le graphène 2 en conséquence. Nous changeons la nature des ions de l'électrolyte 10, sa concentration, son pH et même la densité de charge à l'interface électrolyte 10 / couche de passivation 8 par dépôt local des molécules chargées comme une solution d'ADN (apport de charge négative) ou une solution de poly-L-Lysine (chargée positivement). Nous avons pu observer le décalage du point de Dirac en changeant certaines propriétés de l'électrolyte 10.

L'électrolyte 10 utilisée est de préférence une solution de KCI ou NaCl.

L'électrolyte 10 (typiquement KCI ou NaCl) a typiquement une concentration supérieure à 10 µM de préférence supérieure à 10 mM de préférence supérieure à 20 mM et/ou inférieure à 80 mM de préférence inférieure à 50 mM.

Cela permet de maintenir le point de Dirac aussi proche que possible de 0 Volts.

L'électrolyte 10 utilisé a de préférence un pH supérieur à 6 et/ou inférieur à 8. On ajuste par exemple le pH d'une solution de KCI avec du KOH.

Cela permet de maintenir le point de Dirac aussi proche que possible de 0 Volts.

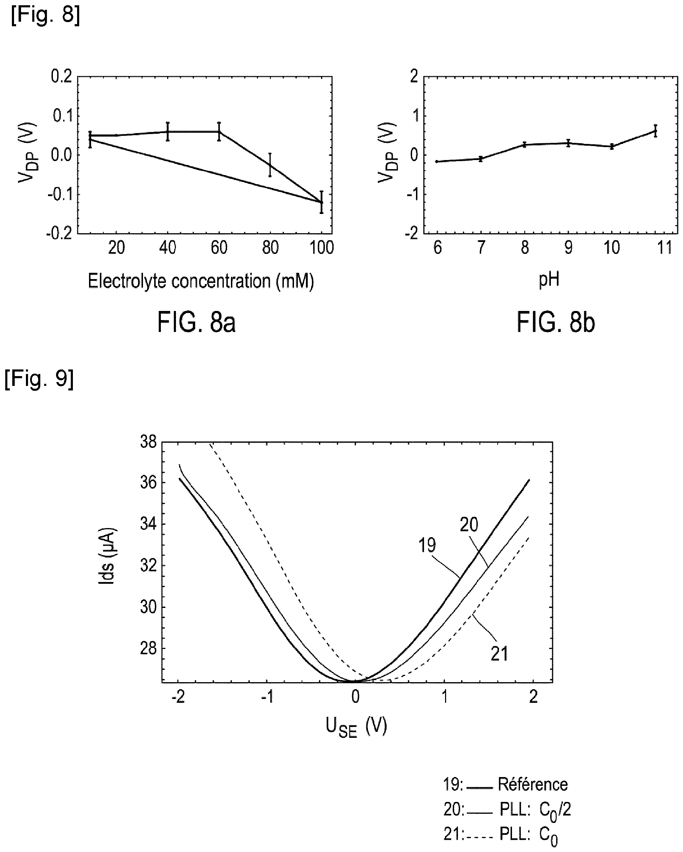

Sur la figure 8 :
- la figure 8 a) illustre, pour le dispositif 1, la variation de la tension au point de Dirac V_{DP} pour une zone de mesure en fonction de la concentration de l'électrolyte 10 de KCI.; Des solutions de KCI de différentes concentrations sont préparées ; l'électrode 14 et le réservoir 9 sont rincés à l'eau déionisée puis séchés à l'air comprimé après chaque point de mesure et
- la figure 8 b) illustre, pour le dispositif 1, la variation de la tension au point de Dirac V_{DP} pour une zone de mesure en fonction du pH de l'électrolyte 10 de 25mM de KCI dont le pH est ajusté par une solution basique de KOH.

Les figures 9, 10, 11a), 11b), 12a), 12b), 13, 14, 15a), 15b), 16a), 17b), et 18 illustrent une mesure de courant électrique Ids circulant entre la source 71 et un drain 72 (reliés par une zone de mesure, chaque zone de mesure correspondant à un « transistor ») du dispositif 1 selon l'invention en fonction d'une tension électrique imposée U_{SE} entre la source 7,71 et l'électrode 14 plongée dans l'électrolyte.

En référence à la Figure 9, la polylysine est un polymère largement utilisé dans la technique de fixation des molécules d'ADN sur les biopuces. Elle est positivement chargée et adhère facilement à des surfaces chargées négativement comme l'oxyde de silicium. On dépose et on vérifie l'effet de la couche de polymère 13 de la manière suivante. On fait une mesure de référence (courbe 19) sur le dispositif 1 bien nettoyé et on l'incube dans une solution de polylysine de concentration Co /2 (avec Co = 85 µM) pendant 30minutespuis on laisse sécher pendant une journée à température ambiante avant de réaliser une mesure de détection (courbe 20). Le dispositif 1 est à nouveau incubé dans une solution de polylysine de concentration Co puis laissé séché de nouveau pour une deuxième mesure de détection (courbe 21).

Nous observons un décalage positif du point de Dirac et proportionnel à la concentration de la solution de polylysine. Un apport de charge positive produit un décalage positif du point de Dirac. Cette réponse est proportionnelle à la concentration de la polylysine. La sensibilité du dispositif 1 est typiquement de 5 mV/µM à 30 ± 5 mV/ µM.

Les différentes séquences utilisées dans la présente description sont détaillées dans la table ci-dessous.

**[Table 1]**

| Nom | Séquence | Fonctionnalisation | Nombre de bases | Complémentaire de |
|---|---|---|---|---|
| Ars3 | [5']CCGCGAACTGACTCTCCGCC[3'] | non | 20 | Ars3sens |
| Ars3sens | [5']GGC GGA GAG TCA GTT CGC GG[3'] | Cy3 ou Cy5 | 20 | Ars3 |
| Ars5 | [5']CAG GCG GCA GGG CTG ACG TT[3'] | non | 20 | Ars5sens |
| Ars5sens | [5']AAC GTC AGC CCT GCC GCC TG[3'] | Cy3 ou Cy5 | 20 | Ars5 |
| Ars7 | [5']TAT AGC GTA CGA ACT CCA GC[3'] | non | 20 | Ars7sens |
| Ars7sens | [5']GCT GGA GTT CGT ACG CTA TA[3'] | Cy3 ou Cy5 | 20 | Ars7 |
| Lprime | [5']GGG TTT TGC TAT CAC GTT GTG[3'] | non | 21 | - |

En référence à la Figure 10, après l'adsorption de la polylysine, nous immobilisons des oligonucléotides sondes (1µM) sur le dispositif 1 (dans le réservoir 9) par dépôt local d'une goutte de l'ordre de 0,1 µL. Après 45 minutes de séchage on fait une mesure de détection en prenant comme référence la mesure de détection de la polylysine. La fixation des sondes qui est synonyme d'un apport de charge négative induit un décalage négatif du point de Dirac. Un apport de charge négative produit un décalage négatif. Pour ce transistor on peut voir que la totalité de la polylysine a été fixée par les sondes ce qui explique les décalages opposés mais de même ordre de grandeur qu'on observe. Pour l'ensemble des zones de mesure (chaque zone de mesure correspondant à un « transistor ») du dispositif 1, l'adsorption de la polylysine a induit un décalage de 1,003 ± 0,2 V. La fixation des sondes a induit un décalage de -0,9 ± 0,3 V. Ceci parait logique puisqu'on peut ne pas fixer la totalité de la polylysine et explique l'importance des différents blocages (chimique et physique) réalisés pour éviter les adsorptions non spécifiques des cibles lors de l'hybridation. La courbe 22 est une courbe de référence avant dépôt de polylysine, la courbe 23 est une courbe de mesure après dépôt de polylysine mais avant dépôt de la sonde et la courbe 24 est une courbe de mesure après dépôt de la sonde.

En référence à la figure 11, après la fixation des sondes, on procède à la neutralisation des résidus de polylysine sur les transistors étudiés par :
- Blocage chimique : C'est une étape qui consiste à incuber le dispositif 1 pendant 30 minute(s) dans une solution de d'anhydride acétique.
- Blocage physique : le dispositif 1 (aussi appelé « puce ») est, après le blocage chimique, ensuite incubé pendant 30 minutes dans une solution contenant des oligonucléotides de séquence différente de celle des cibles complémentaires des sondes préalablement fixées.

On fait alors une mesure de référence (courbes 25 et 26) avant de passer à l'hybridation en incubant le dispositif 1 dans une solution contenant les cibles complémentaires des sondes.

Après hybridation, on fait une mesure de détection (courbes 27 et 28).

La figure 11 illustre la réponse de deux zones de mesures différentes après hybridation. A gauche (11a) ), une zone de mesure sur laquelle on a fixé des sondes, on observe un décalage de -20 mV. A droite (11b) ) une zone de mesure témoin sur laquelle on n' a pas fixé les sondes. La concentration en cible est de 10 nM et Vds = 10 mV.

L'ordre de grandeur du décalage sur le dispositif 1 de la figure 11a) par rapport à la concentration en cible et la non sensibilité du transistor témoin de la figure 11b) nous permet de conclure que le décalage que nous observons est réellement dû à l'hybridation entre les sondes et les cibles.

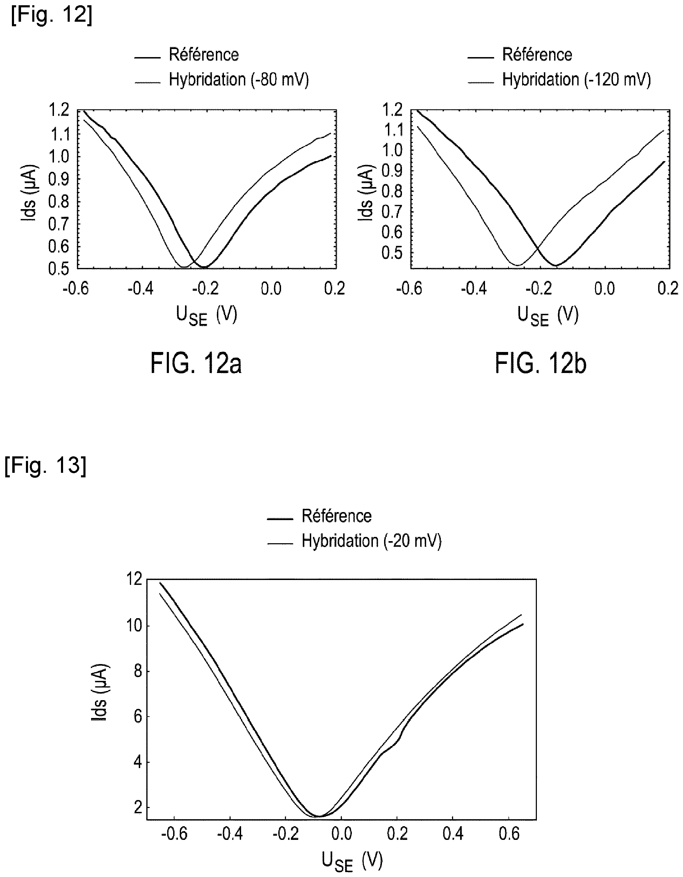

En référence à la figure 12, l'expérience de l'hybridation a été faite dans les mêmes conditions que précédemment en variant la concentration des cibles. Lors de la première expérience, la concentration des cibles est 20 nM et lors de la deuxième nous avons doublé la concentration. La figure 12 illustre la réponse d'un même transistor suite à la variation de la concentration des cibles. A gauche ( 12 a) ) l'hybridation est faite avec une concentration de 20 nM et à droite ( 12b) ), la concentration est de 40 nM ; les autres conditions expérimentales étant maintenues fixes (Blocage chimique 30 minutes, Blocage physique 30 minutes, électrolyte de mesure KCI (25 mM), durée d'hybridation 30 minutes) ; on a Vds = 10 mV. Avec cette expérience nous avons remarqué que les transistors sont sensibles à la variation de la concentration des cibles. Les décalages observés sur les dispositifs 1 étudiés sont proportionnels à la concentration des cibles.

En référence à la figure 13, nous avons cherché à savoir quelle peut être la plus faible concentration des cibles que l'on peut détecter. L'expérience d'hybridation est alors faite dans cette optique en utilisant cette fois ci une très faible concentration des cibles (1 nM) en gardant les autres conditions expérimentales inchangées. On a dans ce cas Vds = 50 mV. Sur la base des résultats de cette expérience nous pouvons conclure que la sensibilité du dispositif 1 est de l'ordre de -20 mV/ nM.

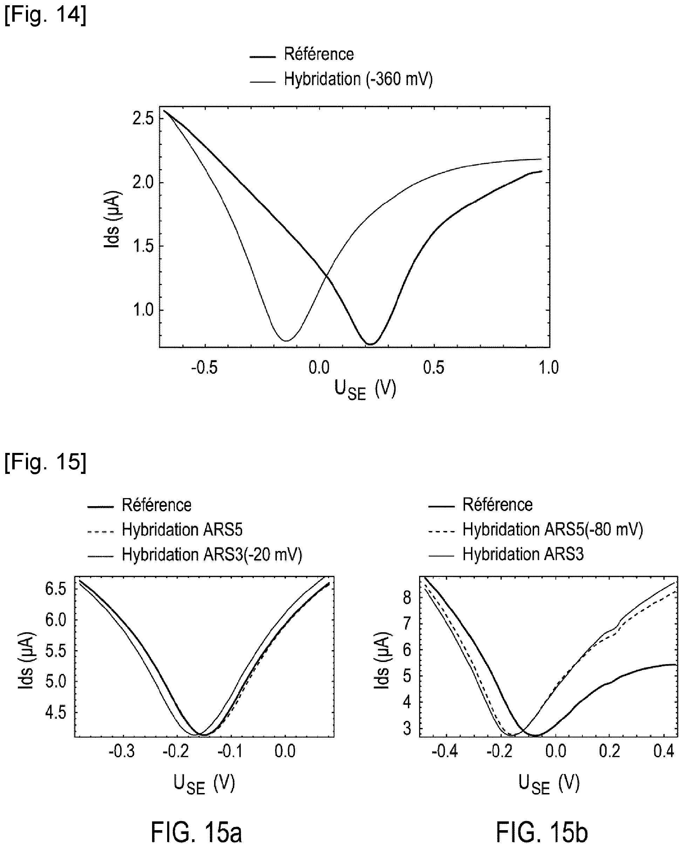

La concentration de l'électrolyte de mesure dans les expériences précédentes est généralement de 25 mM. En référence à la Figure 14, nous avons alors essayé de faire l'hybridation à une concentration beaucoup plus faible. L'expérience de l'hybridation est alors refaite en utilisant un électrolyte de KCI à 10 mM. La figure 14 illustre la réponse d'un transistor lors d'une hybridation à bas sel (KCL 10 mM). La concentration des cibles est de 20 nM. Les autres conditions expérimentales sont restées inchangées. On a Vds = 10 mV. Nous avons observé des décalages plus importants lors de cette expérience sur tous les transistors étudiés. La concentration en sel joue sur l'intensité du signal détecté. Plus l'électrolyte est chargé en sel plus les phénomènes d'écrantage sont observés et diminuent le signal électrique.

Lors des expériences précédentes nous avons immobilisé des sondes de même nature soit ARS3 ou ARS5. Dans la nouvelle expérience illustrée en Figure 15 nous avons déposé dans le réservoir 9 trois gouttes de trois espèces différentes sur le réseau de transistors: ARS3, ARS5 et ARS7 de concentration 1 µM. Après les séries de blocage chimique et physique, nous avons procédé à deux réactions d'hybridation. La première avec les cibles ARS5sensCy5 complémentaires de ARS5 et la deuxième avec les ARS3sensCy3 complémentaires de ARS3. La partie du réseau recouverte par ARS7 étant restée témoin. Nous avons remarqué que certains transistors recouverts de ARS3 et ARS5 sont restés insensibles lors des deux séries d'hybridation en plus de ceux de ARS7 (ce qui est normal pour ces derniers). Par ailleurs, des transistors ont réagi sélectivement lors des hybridations comme on peut le voir sur les figures suivantes. On peut aussi voir que les décalages sont moins prononcés lors de la deuxième hybridation. La figure 15 illustre la réponse de deux transistors lors des hybridations sens et anti-sens : à gauche (15a) ) la détection de ARS3 et à droite (15b) ) la détection de ARS5. Le petit décalage que nous observons lors de l'hybridation de ARS3 sur le dispositif 1 de droite serait dû à l'hybridation non spécifique entre ARS3sensCy3 et ARS5. On a Vds = 90mV. Cette expérience vient prouver que les décalages que nous observons lors de l'hybridation ne sont pas aléatoires. Des transistors réagissent sélectivement vis-à-vis des cibles complémentaires.

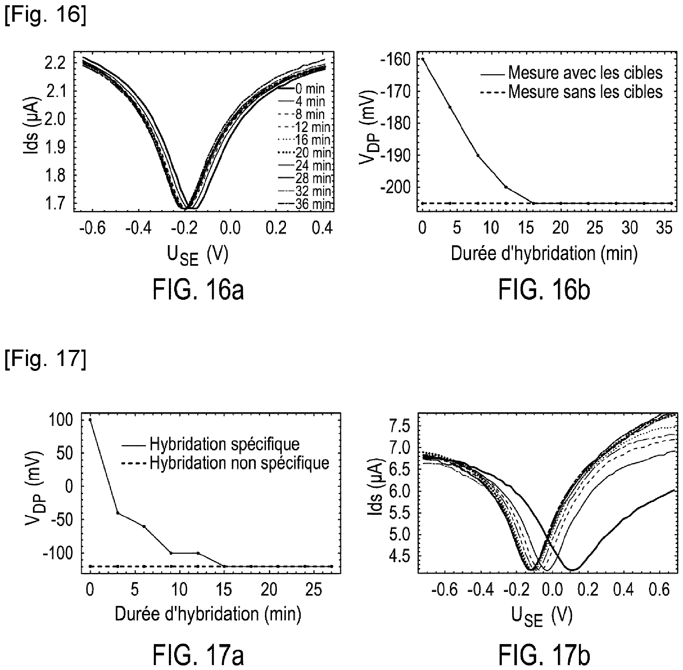

Afin de voir comment se comportent les transistors en temps réel de l'hybridation, nous avons lancé une série de 10 mesures à intervalles de temps 4 minutes. Le tampon d'hybridation est du 0,1XPBS. A la fin de l'expérience, un test de contrôle est fait avec le même tampon sans les cibles. Nous avons observé des dérives au cours des 16 premières minutes. Ces dérives sont assez marquées au début, diminuent avec le temps et finissent par s'estomper. Par ailleurs ces dérives ne sont jamais apparues lors des mesures sans les cibles ou en présence des cibles non complémentaires. La Figure 16 illustre le comportement d'un transistor en temps réel lors de l'hybridation. A gauche ( 16a) ), l'évolution de la caractéristique entre t= 0minute et t = 36 minutes de droite à gauche, et à droite ( 16b) ), les décalage du point de Dirac avec les cibles et sans les cibles; on a Vds = 50mV. La Figure 17 illustre le comportement d'un transistor en temps réel lors de l'hybridation. A droite ( 17b) ), l'évolution de la caractéristique entre t= 0 minute et t = 36 minutes de droite à gauche, et à gauche ( 17a) ), les décalages du point de Dirac avec les cibles complémentaires et avec les cibles non complémentaires ; on a Vds = 50mV. Avec cette expérience nous avons pu observer l'évolution du point de Dirac en temps réel lors de l'hybridation. La durée de l'hybridation est estimée à 16 minutes vu qu'au-delà de cette période aucun décalage n'est observé. On peut alors calculer la vitesse de déplacement du point de Dirac en calculant la pente de la courbe d'hybridation spécifique en figure 16b) ou 17a). Cette vitesse est de -2,8 mV/ minute. Une autre expérience lors de laquelle on a utilisé les cibles non complémentaires après l'hybridation spécifique a donné le même résultat. Le déplacement du point de Dirac est estimé à -14,7mV/min. Cette deuxième expérience confirme que le signal détecté n'est pas dû à l'adsorption non spécifique des cibles. Il s'agit bien d'une réaction entre les sondes et leurs complémentaires.

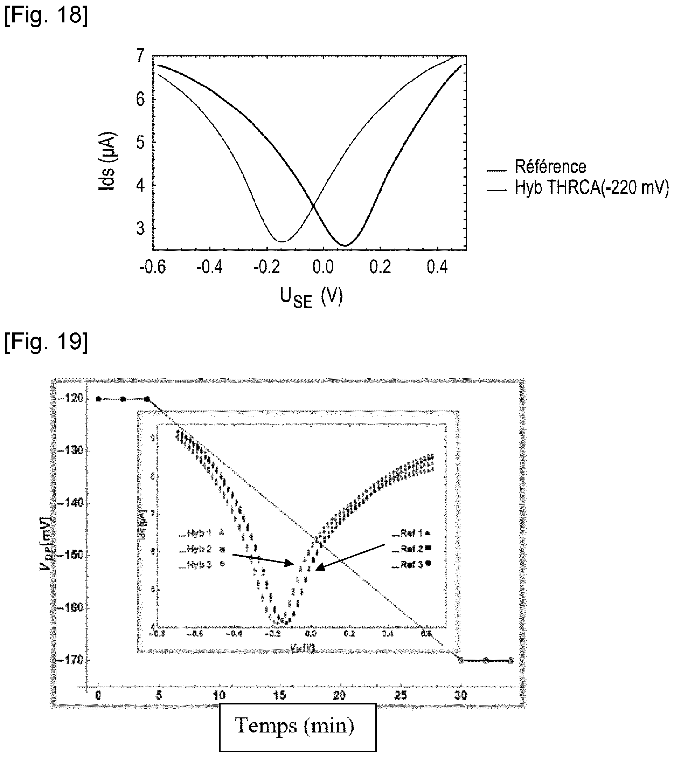

Enfin, les produits THRCA (pour « Tagged Hyperbranched Rolling Circle Amplification ») tels que décrits dans WO2011/018774 sont des molécules complexes constituées d'une partie simple brin dont la séquence peut être celle de ARS3 ou ARS5 et une partie double brin qui peut être reproduite de façon périodique. Ce sont des molécules qui apportent beaucoup de charges sur le dispositif 1. L'hybridation de ces molécules est un peu plus compliquée que celle des oligonucléotides. La partie double brin étant plus longue, ces molécules migrent plus lentement en solution que les oligonucléotides ce qui demande un temps d'hybridation plus long. D'après des résultats obtenus en détection par fluorescence, il faut un temps de blocage chimique plus long pour une hybridation spécifique des produits THRCA que pour une hybridiation spécifique des oligonucleotides. Un autre paramètre qu'on a pu contrôler lors de la détection par fluorescence était la température. L'hybridation à une température de 50°C avait considérablement réduit les hybridations non spécifiques. Des sondes ARS3 de concentration 2µM ont été fixés par la polylysine comme dans un processus normal d'hybridation des oligonucléotides. Le dispositif 1 est alors séché pendant 45 minutes, incubé dans de l'anhydride acétique pendant trois heures et rincé proprement à l'eau déionisée. Ce blocage reste nécessaire même si nous ne sommes pas en analyse de rapport signal sur bruit. En effet les inventeurs ont remarqué que toute la polylysine n'est pas fixée par les sondes et, en plus, il faut bloquer la polylysine environnante pour éviter la réaction préférentielle c'est-à-dire l'attraction en les charges positives de la polylysine et les charges négatives des molécules THRCA. Une solution des cibles (THRCA) est alors préparée (12 µL de produit dans 1 mL de PBS final) et hybridée pendant une heure. La figure 18 illustre la mesure avant (référence) et après hybridation. Les produits THRCA sont des molécules longues et donc très chargées. Ceci explique l'intensité forte du signal donné par les transistors après l'hybridation.

L'expérience présentée sur la figure 19 vise à déterminer une concentration de cible très basse pouvant être détectée selon l'invention. Puisque la sensibilité des transistors diminue lors d'une utilisation répétée, l'expérience a été réalisée avec un réseau de transistors à effet de champs à base de graphène (aussi appelé GFET) fraîchement préparé appartenant à un dispositif selon l'invention 1 tel que précédemment illustré, pour avoir une sensibilité élevée.

Les étapes de blocage évitent une adsorption non spécifique et empêchent de perdre une quantité importante de cible à faible concentration dans cette mesure.

La concentration des cibles a été fixée à 10 fM. Après incubation de la puce 1 avec des cibles pendant 30 min, la puce 1 a été rincée puis trois mesures de détection ont été effectuées toutes les deux minutes. L'amplitude du signal observé (-50 mV, décalage vers le bas de -120mV à -170mV) a fortement dépassé la dérive expérimentale (la dérive n'est pas détectée sur l'échelle de temps, comme l'illustrent trois points de données avant et trois points de données après l'hybridation). Cela suggère la possibilité d'obtenir une détection en dessous de la plage femtomolaire.

Un GFET voisin du dispositif 1 a été mesuré en parallèle et a également détecté une hybridation spécifique, bien qu'avec un décalage de plus petite amplitude (-21mV).

Nous avons répété l'expérience avec un autre réseau GFET selon l'invention fraîchement préparé. Dans ce cas, quatre transistors ont été enregistrés et tous ont détecté l'hybridation à une concentration cible de 10 fM (-19, -18, -25, -22mV).

En utilisant un autre GFET 1 fraîchement préparé, nous avons ensuite tenté de détecter l'hybridation à une concentration cible réduite de dix fois (1fM). Nous avons mesuré cinq transistors du réseau et observé un très petit signal d'hybridation pour deux d'entre eux (des décalages d'environ -5 et -6 mV), mais sinon nous n'avons pas observé un décalage qui dépassait clairement l'incertitude de mesure d'environ 2 mV. Pour les mesures présentées ici, l'incertitude de mesure est principalement donnée par la précision de la détermination de la tension du point Dirac, qui dépend de la taille de pas des enregistrements de tension U_{SE} discrets (ici 10 mV), et de la largeur du minimum I_{DS} (qui dépend de la mobilité des porteurs de charges).

Ainsi, nous avons obtenu une détection reproductible de l'hybridation avec des oligonucléotides d'ADN à 20 bases à une concentration de 10 fM. L'hybridation d'une seule molécule cible correspond à la formation de 20 paires de bases d'ADN. La limite de détection de 10 fM de molécules cibles correspond ainsi à une limite de détection de 200 fM en termes de paires de bases d'ADN individuelles. Nous attribuons la haute sensibilité à la bonne capacité de transduction des réseaux GFET selon l'invention rendue possible par deux éléments originaux.

Premièrement, les décalages du point de Dirac dus à l'incorporation d'impuretés lors de la fabrication d'un dispositif selon l'état de l'art nuisent à la sensibilité. Nous avons réduit ce décalage grâce à l'invention par un processus de fabrication innovant qui protège le graphène lors de la structuration photolithographique. Les GFET tels que fabriqués selon l'invention présentent une mobilité élevée et l'emplacement du point Dirac est bien défini, comme le montrent les caractéristiques de courant-tension en forme de V raide. En effet, de petits décalages de tension de seuil induits par l'hybridation d'ADN sont plus facilement résolus si le point de Dirac se manifeste comme un pic aigu.

Deuxièmement, nous avons utilisé une fine couche de protection qui sépare le graphène de la solution d'électrolyte lors de l'hybridation de l'ADN et de la mesure électronique. Cette couche diélectrique permette d'atteindre à la fois la sensibilité et la stabilité de la tension de seuil sous l'électrolyte (c'est-à-dire une très petite dérive résiduelle). Nous avons trouvé une bonne solution pour la mise en oeuvre de cette couche : une double couche déposée par ALD et PECVD.

Le dispositif 1 selon l'invention présente des canaux en graphène recouverts d'une fine couche isolante. Cette couche confère selon l'invention une stabilité dans le temps aux caractéristiques du dispositif 1 et évite la contamination chimique du graphène lors du contact avec les solutions d'électrolyte.

La surface en contact avec l'électrolyte est du SiOz, comme pour les réseaux de silicium utilisés selon l'état de l'art. De plus, les compositions de sel des solutions d'électrolyte utilisées ici sont similaires aux solutions utilisées selon l'état de l'art. Comme prévu, les signes des changements de tension de seuil observés selon l'invention (lors de l'adsorption de PLL, de l'adsorption d'ADN et de l'hybridation d'ADN) coïncident avec ceux décrits pour les dispositifs en silicium selon l'état de l'art.

Remarquablement, nos dispositifs GFET 1 selon l'invention présentent de très petites dérives de tension de seuil (inférieures à 1 mV par heure, voir Figure 19), beaucoup plus petites que celles observées avec les dispositifs au silicium selon l'état de l'art (environ 0,2-1 mV / min, voir pour exemple, J. Fritz, C. Emily, G. Suzanne, S. Peter and M. Scott, "Electronic détection of DNA by its intrinsic molecular charge," PNAS, vol. 99, no. 22, pp. 14142-14146, 2002. ; K. Malpartida-Cardenas, N. Miscourides and J. Rodriguez-Manzano, "Quantitative and rapid Plasmodium falciparum malaria diagnosis and artemisinin-resistance détection using a CMOS Lab-on-Chip platform," Biosens. Bioelectron, vol. 11, no. 145, pp. 0956-5663, 2019; C. Perréard, A. Blin and U. Bockelmann, "Threshold voltage drift of FET sensor arrays with different gate insulators," Sens. Actuator B-Chem, no. 185, pp. 282-286, 2013.).

De plus, les amplitudes des signaux électroniques d'hybridation d'ADN sont d'environ 100 mV pour nos réseaux GFET selon l'invention, dépassant fortement les amplitudes typiques de 3 à 10 mV des réseaux FET au silicium selon l'état de l'art (voir A. Blin, I. Cissé and U. Bockelmann, "Electronic hybridization détection in microarray format and DNA genotyping," Sci. Rep, vol. 4, p. 4194, 2014. ; J. Fritz, C. Emily, G. Suzanne, S. Peter and M. Scott, "Electronic détection of DNA by its intrinsic molecular charge," PNAS, vol. 99, no. 22, pp. 14142-14146, 2002. ; C. Gentil, G. Philippin and U. Bockelmann, "Signal enhancement in electronic détection of DNA hybridization," Phys. Rev. E, vol. 75, no. 1, p. 011926, 2007. ).

Selon l'invention, comme illustré sur la figure 16, la combinaison d'une petite dérive et d'une amplitude de signal élevée facilite grandement la détection d'hybridation d'ADN en temps réel par rapport aux dispositifs en silicium selon l'état de l'art.

Bien sûr, l'invention n'est pas limitée aux exemples qui viennent d'être décrits et de nombreux aménagements peuvent être apportés à ces exemples sans sortir du cadre de l'invention.

Par exemple, dans une variante de l'invention :
- le procédé d'utilisation d'un dispositif selon l'invention ou obtenu par un procédé de fabrication selon l'invention, comprend une étape de détection d'une activité électrique d'au moins une cellule biologique sur ou au-dessus de la couche conductrice ou de la couche de passivation, comme par exemple d'au moins un neurone, et/ou
- la première lithographie de la couche 5 et la deuxième lithographie de la couche 6 peuvent être deux lithographies positives ou deux lithographies négatives, et/ou
- après le dépôt de l'au moins une électrode 7, on peut conserver la couche de protection 4 de manière à la conserver entre la couche 2 et la couche 8 (81, 82).

## Revendications

1. Procédé de fabrication d'un dispositif (1) comprenant :
- une fourniture d'une couche conductrice (2) entre un substrat (3) et une couche de protection (4),
- un dépôt d'une première couche de résine (5) de lithographie au-dessus ou sur la couche de protection (4) de sorte que la couche de protection (4) soit comprise entre la couche conductrice (2) et la première couche de résine (5),
- une première lithographie comprenant :
a) un retrait, dans au moins une zone de retrait (51) de la première lithographie, de la superposition de la première couche de résine (5), de la couche de protection (4) et de la couche conductrice (2), et
b) une conservation, dans au moins une zone de conservation (52) de la première lithographie, de la superposition de la première couche de résine (5), de la couche de protection (4) et de la couche conductrice (2), et
- un dépôt, au moins sur l'au moins une zone de conservation (52) de la première lithographie, de préférence sur l'au moins une zone de retrait (51) et sur l'au moins une zone de conservation (52) de la première lithographie, d'une deuxième couche de résine (6) de lithographie sans retirer la première couche de résine (5) de l'au moins une zone de conservation (52) de la première lithographie, et
- une deuxième lithographie comprenant :
a) un retrait, dans au moins une zone de retrait (61) de la deuxième lithographie, de la superposition de la deuxième résine (6) , de la première résine (5), de la couche de protection (4) mais pas de la couche conductrice (2), et
b) une conservation, dans au moins une zone de conservation (62) de la deuxième lithographie, de la superposition de la deuxième résine (6), de la première résine (5), de la couche de protection (4) et de la couche conductrice (2).

2. Procédé selon la revendication 1, **caractérisé en ce que** la couche conductrice (2) comprend ou consiste en conducteur bidimensionnel ayant une mobilité supérieure ou égale à 1000 cm².V⁻¹s⁻¹, consistant de préférence en une couche de graphène, de silicène, de disulfite de molybdène (MoS₂), de germanène, de phosphorène, de stannène, ou de borophène .

3. Procédé selon l'une quelconque des revendications précédentes, **caractérisé en ce que** la première lithographie est une lithographie respectivement positive ou négative et **en ce que** la deuxième lithographie est une lithographie respectivement négative ou positive, la première couche de résine (5) et la deuxième couche de résine (6) étant constituées d'une seule et même résine compatible pour une lithographie positive et pour une lithographie négative.

4. Procédé selon l'une quelconque des revendications précédentes, **caractérisé en ce que** la résine de la première couche de résine (5) et la résine de la deuxième couche de résine (6) sont agencées pour réagir, lors d'une étape de lithographie :
- au même développeur, et/ou
- à la même durée, puissance et longueur d'onde d'un rayonnement de préférence ultra violet.

5. Procédé selon l'une quelconque des revendications précédentes, **caractérisé en ce que** le substrat (3) comprend ou consiste en du silicium et/ou de l'oxyde de silicium.

6. Procédé selon l'une quelconque des revendications précédentes, **caractérisé en ce que** la couche de protection (4) comprend ou consiste en de l'aluminium oxydé, de préférence de l'alumine Al₂O₃.

7. Procédé selon l'une quelconque des revendications précédentes, **caractérisé en ce qu'**il comprend en outre, dans l'au moins une zone de retrait (61) de la deuxième lithographie, un dépôt d'au moins une électrode (7) en contact électrique avec la couche conductrice (2).

8. Procédé selon la revendication précédente, **caractérisé en ce qu'**il comprend en outre, après le dépôt de l'au moins une électrode (7), un retrait, dans l'au moins une zone de conservation (62) de la deuxième lithographie, de la superposition de la deuxième résine (6), de la première résine (5) mais pas de la couche conductrice (2).

9. Procédé selon la revendication précédente, **caractérisé en ce qu'**il comprend en outre, après le retrait selon la revendication précédente, un dépôt d'une couche de passivation (8) en contact avec la couche conductrice (2) de l'au moins une zone de conservation (62) de la deuxième lithographie et en contact avec au moins une partie de chaque électrode (7).

10. Procédé selon la revendication précédente, **caractérisé en ce que** la couche de passivation (8) comprend une superposition de deux matières différentes.

11. Procédé selon la revendication précédente, **caractérisé en ce que** la couche de passivation (8) comprend une superposition :
- de Al₂O₃ en contact avec la couche conductrice (2), puis
- d'oxyde de silicium en contact avec l' Al₂O₃ de la couche de passivation, de sorte que de l' Al₂O₃ de la couche de passivation soit compris entre la couche conductrice (2) et l'oxyde de silicium de la couche de passivation.

12. Procédé selon la revendication précédente, **caractérisé en ce que** la couche de passivation (8) à un champ de claquage supérieur ou égal à 10⁶ V.m⁻¹.

13. Dispositif, comprenant :
- un substrat (3),
- une couche conductrice (2) répartie en au moins une zone de mesure,
- au moins deux électrodes (7) distinctes en contact avec la couche conductrice (2) **caractérisé en ce que** :
- la couche conductrice (2) consiste en une couche d'un conducteur bidimensionnel ayant un niveau de dopage inférieur à 10¹¹ impuretés par cm², et/ou un décalage du point de Dirac par rapport à 0 volt inférieur à 1 Volt en valeur absolue,
- il comprend en outre une couche de passivation (8) ayant un champ de claquage supérieur ou égal à 10⁶ V.m⁻¹ et recouvrant la couche conductrice (2) et au moins une partie de chaque électrode (7), de sorte que la couche conductrice (2) et au moins une partie de chaque électrode (7) se trouve entre le substrat (3) et la couche de passivation (8).

14. Dispositif selon la revendication 13, **caractérisé en ce que** la couche conductrice (2) est répartie en plusieurs zones de mesure distinctes.

15. Dispositif selon la revendication 14, **caractérisé en ce que** les électrodes (7) comprennent une source commune (71) en contact avec toutes les zones de mesure et un drain (72) par zone de mesure, chaque drain (72) n'étant en contact qu'avec une seule zone de mesure.

16. Dispositif selon l'une quelconque des revendications 13 à 15, **caractérisé en ce qu'**il comprend en outre un réservoir (9) agencé pour accueillir un électrolyte (10) au-dessus de la couche conductrice (2), de préférence de sorte que l'électrolyte (10) soit en contact avec la couche de passivation (8) ou avec la couche conductrice (2).

17. Dispositif selon la revendication 16, **caractérisé en ce qu'**il comprend des moyens (11) pour mesurer et/ou imposer une tension (Vse) et/ou une intensité électrique entre l'électrolyte (10) et une électrode source (71).

18. Dispositif selon l'une quelconque des revendications 13 à 17, **caractérisé en ce qu'**il comprend des moyens (11) pour mesurer et/ou imposer une tension (V_{SG}) et/ou une intensité électrique entre une grille (3) et une électrode source (71).

19. Dispositif selon l'une quelconque des revendications 13 à 18, **caractérisé en ce qu'**il comprend des moyens (12) pour mesurer et/ou imposer une tension (Vds) et/ou une intensité électrique (Ids) entre une électrode source (71) et une électrode drain (72).

20. Dispositif selon l'une quelconque des revendications 13 à 19, **caractérisé en ce qu'**il comprend une couche (13) de polymère et/ou de nucléotides au-dessus de l'au moins une zone de mesure, en contact avec la couche conductrice (2) ou en contact avec la couche de passivation (8).

21. Procédé d'utilisation d'un dispositif selon l'une quelconque des revendications 13 à 19 ou obtenu par un procédé selon l'une quelconque des revendications 1 à 12, comprenant :
- une étape de détection de fixation de cellules biologiques ou d'atomes ou de molécules sur ou au-dessus de la couche conductrice (2) ou de la couche de passivation (8), et/ou
- une étape d'une détection d'une activité électrique d'au moins une cellule biologique, sur ou au-dessus de la couche conductrice ou de la couche de passivation.

22. Procédé selon la revendication 21, **caractérisé en ce que** la fixation est une fixation de cellules biologiques ou d'atomes ou de molécules sur une couche de polymère et/ou de nucléotides fixée sur la couche conductrice (2) ou sur la couche de passivation (8).

23. Procédé selon la revendication 21 ou 22, **caractérisé en ce que** la fixation est une hybridation de nucléotides sur une couche (13) de polymère et de nucléotides située au-dessus de l'au moins une zone de mesure, et en contact avec la couche conductrice (2) ou en contact avec la couche de passivation (8).

## Patentansprüche

1. Verfahren zur Herstellung einer Vorrichtung (1), mit den Schritten:
- Einbringen einer leitfähigen Schicht (2) zwischen einem Substrat (3) und einer Schutzschicht (4),
- Abscheiden einer ersten Lithographie-Harzschicht (5) über oder auf der Schutzschicht (4), so dass die Schutzschicht (4) zwischen der leitfähigen Schicht (2) und der ersten Harzschicht (5) enthalten ist,
- erster Lithographie-Druck, der die folgenden Schritte umfasst:
a) Entfernen der Überlagerung der ersten Harzschicht (5), der Schutzschicht (4) und der leitfähigen Schicht (2) in zumindest einem Entfernungsbereich (51) des ersten Lithographie-Drucks, und
b) Bewahren der Überlagerung der ersten Harzschicht (5), der Schutzschicht (4) und der leitfähigen Schicht (2) in zumindest einem Erhaltungsbereich (52) des ersten Lithographie-Drucks, und
- Abscheiden einer zweiten Lithographie-Harzschicht (6) zumindest auf dem zumindest einen Erhaltungsbereich (52) des ersten Lithographie-Drucks, vorzugsweise auf dem zumindest einen Entfernungsbereich (51) und dem zumindest einen Erhaltungsbereich (52) des ersten Lithographie-Drucks, ohne dabei die erste Harzschicht (5) von dem zumindest einen Erhaltungsbereich (52) des ersten Lithographie-Drucks zu entfernen, und
- zweiter Lithographie-Druck, der die folgenden Schritte umfasst:
a) Entfernen der Überlagerung des zweiten Harzes (6), des ersten Harzes (5), der Schutzschicht (4), jedoch nicht der leitfähigen Schicht (2), in zumindest einem Entfernungsbereich (61) des zweiten Lithographie-Drucks, und
b) Bewahren der Überlagerung des zweiten Harzes (6), des ersten Harzes (5), der Schutzschicht (4) und der leitfähigen Schicht (2) in zumindest einem Erhaltungsbereich (62) des zweiten Lithographie-Drucks.

2. Verfahren nach Anspruch 1, **dadurch gekennzeichnet, dass** die leitfähige Schicht (2) einen zweidimensionalen Leiter mit einer Mobilität von mehr oder gleich 1000 cm²·V⁻¹s⁻¹ enthält oder daraus besteht, der vorzugsweise aus einer Schicht aus Graphen, Silicen, Molybdändisulfid (MoS₂), Germanen, Phosphoren, Stanen oder Borophen besteht.

3. Verfahren nach einem der vorhergehenden Ansprüche, **dadurch gekennzeichnet, dass** der erste Lithographie-Druck ein positiver bzw. negativer Lithographie-Druck ist und dass der zweite Lithographie-Druck ein negativer bzw. positiver Lithografie-Druck ist, wobei die erste Harzschicht (5) und die zweite Harzschicht (6) aus ein und demselben Harz bestehen, das für Positivlithografie und für Negativlithografie geeignet ist.

4. Verfahren nach einem der vorhergehenden Ansprüche, **dadurch gekennzeichnet, dass** das Harz der ersten Harzschicht (5) und das Harz der zweiten Harzschicht (6) dazu vorgesehen sind, in einem Lithographie-Schritt
- auf den gleichen Entwickler und/oder
- mit der gleichen Zeitdauer, Leistung und Wellenlänge einer vorzugsweise ultravioletten Strahlung zu reagieren.

5. Verfahren nach einem der vorhergehenden Ansprüche, **dadurch gekennzeichnet, dass** das Substrat (3) Silizium und/oder Siliziumoxid umfasst oder daraus besteht.

6. Verfahren nach einem der vorhergehenden Ansprüche, **dadurch gekennzeichnet, dass** die Schutzschicht (4) oxidiertes Aluminium, vorzugsweise Aluminiumoxid Al₂O₃, umfasst oder daraus besteht.

7. Verfahren nach einem der vorhergehenden Ansprüche, **dadurch gekennzeichnet, dass** es ferner ein Abscheiden zumindest einer Elektrode (7), die in elektrischem Kontakt mit der leitfähigen Schicht (2) steht, in dem zumindest einen Entfernungsbereich (61) des zweiten Lithografie-Drucks umfasst.

8. Verfahren nach dem vorhergehenden Anspruch, **dadurch gekennzeichnet, dass** es ferner nach dem Abscheiden der zumindest einen Elektrode (7) ein Entfernen der Überlagerung des zweiten Harzes (6), des ersten Harzes (5), jedoch nicht der leitfähigen Schicht (2) in dem zumindest einen Erhaltungsbereich (62) des zweiten Lithographie-Drucks umfasst.

9. Verfahren nach dem vorhergehenden Anspruch, **dadurch gekennzeichnet, dass** es nach dem Entfernen gemäß dem vorhergehenden Anspruch ferner ein Abscheiden einer Passivierungsschicht (8) umfasst, die mit der leitfähigen Schicht (2) des zumindest einen Erhaltungsbereichs (62) des zweiten Lithografie-Drucks in Kontakt steht und mit zumindest einem Teil jeder Elektrode (7) in Kontakt steht.

10. Verfahren nach dem vorhergehenden Anspruch, **dadurch gekennzeichnet, dass** die Passivierungsschicht (8) eine Überlagerung von zwei verschiedenen Materialien umfasst.

11. Verfahren nach dem vorhergehenden Anspruch, **dadurch gekennzeichnet, dass** die Passivierungsschicht (8) eine Überlagerung von
- Al₂O₃ in Kontakt mit der leitfähigen Schicht (2) und
- Siliziumoxid in Kontakt mit dem Al₂O₃ der Passivierungsschicht umfasst, so dass das Al₂O₃ der Passivierungsschicht zwischen der leitfähigen Schicht (2) und dem Siliziumoxid der Passivierungsschicht enthalten ist.

12. Verfahren nach dem vorhergehenden Anspruch, **dadurch gekennzeichnet, dass** die Passivierungsschicht (8) ein Durchschlagsfeld von größer oder gleich 10⁶ V.m⁻¹ aufweist.

13. Vorrichtung, die das Folgende aufweist:
- ein Substrat (3),
- eine leitfähige Schicht (2), die in zumindest einen Messbereich aufgeteilt ist,
- zumindest zwei separate Elektroden (7) in Kontakt mit der leitfähigen Schicht (2),
**dadurch gekennzeichnet, dass**
- die leitfähige Schicht (2) aus einer Schicht eines zweidimensionalen Leiters mit einem Dotierungsniveau von weniger als 10¹¹ Verunreinigungen pro cm² und/oder einer Verschiebung des Dirac-Punktes in Bezug auf 0 Volt von weniger als 1 Volt im Absolutwert besteht,
- sie ferner eine Passivierungsschicht (8) umfasst, die ein Durchschlagsfeld von größer oder gleich 10⁶ V.m⁻¹ aufweist und die leitfähige Schicht (2) und zumindest einen Teil jeder Elektrode (7) bedeckt, so dass die leitfähige Schicht (2) und zumindest ein Teil jeder Elektrode (7) zwischen dem Substrat (3) und der Passivierungsschicht (8) liegen.

14. Vorrichtung nach Anspruch 13, **dadurch gekennzeichnet, dass** die leitfähige Schicht (2) in mehrere separate Messbereiche aufgeteilt ist.

15. Vorrichtung nach Anspruch 14, **dadurch gekennzeichnet, dass** die Elektroden (7) eine gemeinsame Source (71), die mit allen Messbereichen in Kontakt steht, und einen Drain (72) pro Messbereich umfassen, wobei jeder Drain (72) nur mit einem einzigen Messbereich in Kontakt steht.

16. Vorrichtung nach einem der Ansprüche 13 bis 15, **dadurch gekennzeichnet, dass** sie ferner ein Reservoir (9) umfasst, das dazu vorgesehen ist, einen Elektrolyten (10) über der leitfähigen Schicht (2) aufzunehmen, vorzugsweise so, dass der Elektrolyt (10) mit der Passivierungsschicht (8) oder mit der leitfähigen Schicht (2) in Kontakt steht.

17. Vorrichtung nach Anspruch 16, **dadurch gekennzeichnet, dass** sie Mittel (11) zum Messen und/oder Aufprägen einer Spannung (Vse) und/oder einer elektrischen Stromstärke zwischen dem Elektrolyten (10) und einer Source-Elektrode (71) umfasst.

18. Vorrichtung nach einem der Ansprüche 13 bis 17, **dadurch gekennzeichnet, dass** sie Mittel (11) zum Messen und/oder Aufprägen einer Spannung (V_{SG}) und/oder einer elektrischen Stromstärke zwischen einem Gate (3) und einer Source-Elektrode (71) umfasst.

19. Vorrichtung nach einem der Ansprüche 13 bis 18, **dadurch gekennzeichnet, dass** sie Mittel (12) zum Messen und/oder Aufprägen einer Spannung (Vds) und/oder einer elektrischen Stromstärke (Ids) zwischen einer Source-Elektrode (71) und einer Drain-Elektrode (72) umfasst.

20. Vorrichtung nach einem der Ansprüche 13 bis 19, **dadurch gekennzeichnet, dass** sie eine Schicht (13) aus Polymer und/oder Nukleotiden über dem zumindest einen Messbereich umfasst, die mit der leitfähigen Schicht (2) in Kontakt steht oder mit der Passivierungsschicht (8) in Kontakt steht.

21. Verfahren zur Verwendung einer Vorrichtung nach einem der Ansprüche 13 bis 19 oder erhalten durch ein Verfahren nach einem der Ansprüche 1 bis 12, umfassend:
- einen Schritt des Erfassens einer Anhaftung von biologischen Zellen oder Atomen oder Molekülen auf oder über der leitfähigen Schicht (2) oder der Passivierungsschicht (8), und/oder
- einen Schritt des Erfassens einer elektrischen Aktivität von zumindest einer biologischen Zelle auf oder über der leitfähigen Schicht oder der Passivierungsschicht.

22. Verfahren nach Anspruch 21, **dadurch gekennzeichnet, dass** die Anhaftung eine Anhaftung von biologischen Zellen oder Atomen oder Molekülen auf einer Schicht aus Polymer und/oder Nukleotiden ist, die auf der leitfähigen Schicht (2) oder der Passivierungsschicht (8) fixiert ist.

23. Verfahren nach Anspruch 21 oder 22, **dadurch gekennzeichnet, dass** die Anhaftung eine Hybridisierung von Nukleotiden auf einer Schicht (13) aus Polymer und Nukleotiden ist, die sich über dem zumindest einen Messbereich befindet und in Kontakt mit der leitfähigen Schicht (2) oder in Kontakt mit der Passivierungsschicht (8) ist.

## Claims

1. Method for producing a device (1) comprising:
- providing a conductive layer (2) between a substrate (3) and a protective layer (4),
- depositing a first lithography resin layer (5) above or on the protective layer (4) such that the protective layer (4) is comprised between the conductive layer (2) and the first resin layer (5),
- a first lithography comprising:
a) removing, in at least one removal area (51) of the first lithography, the superimposition of the first resin layer (5), the protective layer (4) and the conductive layer (2), and
b) preserving, in at least one preservation area (52) of the first lithography, the superimposition of the first resin layer (5), the protective layer (4) and the conductive layer (2), and
- depositing, at least on the at least one preservation area (52) of the first lithography, preferably on the at least one removal area (51) and on the at least one preservation area (52) of the first lithography, a second lithography resin layer (6) without removing the first resin layer (5) of the at least one preservation area (52) of the first lithography, and
- a second lithography comprising:
a) removing, in at least one removal area (61) of the second lithography, the superimposition of the second resin (6), the first resin (5) and the protective layer (4), but not the conductive layer (2),
b) preserving, in at least one preservation area (62) of the second lithography, the superimposition of the second resin (6), the first resin (5), the protective layer (4) and the conductive layer (2).

2. Method according to claim 1, **characterized in that** the conductive layer (2) comprises or consists of a two-dimensional conductor having a mobility greater than or equal to 1,000 cm².V⁻¹s⁻¹, preferably consisting of a layer of graphene, of silicene, of molybdenum disulfide (MoS₂), of germanene, of phosphorene, of stanene, or of borophene.

3. Method according to any one of the preceding claims, **characterized in that** the first lithography is a positive or negative lithography and the second lithography is a negative or positive lithography respectively, the first resin layer (5) and the second resin layer (6) being constituted by one and the same resin compatible for a positive lithography and for a negative lithography.

4. Method according to any one of the preceding claims, **characterized in that** the resin of the first resin layer (5) and the resin of the second resin layer (6) are arranged to react, during a lithography step:
- to the same developer, and/or
- to the same duration, power and wavelength of a preferably ultraviolet radiation.

5. Method according to any one of the preceding claims, **characterized in that** the substrate (3) comprises or consists of silicon and/or silicon oxide.

6. Method according to any one of the preceding claims, **characterized in that** the protective layer (4) comprises or consists of oxidized aluminium, preferably alumina Al₂O₃.

7. Method according to any one of the preceding claims, **characterized in that** it moreover comprises, in the at least one removal area (61) of the second lithography, depositing at least one electrode (7) in electrical contact with the conductive layer (2).

8. Method according to the preceding claim, **characterized in that** it moreover comprises, after the deposition of the at least one electrode (7), removing, in the at least one preservation area (62) of the second lithography, the superimposition of the second resin (6) and the first resin (5), but not the conductive layer (2).

9. Method according to the preceding claim, **characterized in that** it moreover comprises, after the removal according to the preceding claim, depositing a passivation layer (8) in contact with the conductive layer (2) of the at least one preservation area (62) of the second lithography and in contact with at least part of each electrode (7).

10. Method according to the preceding claim, **characterized in that** the passivation layer (8) comprises a superimposition of two different materials.

11. Method according to the preceding claim, **characterized in that** the passivation layer (8) comprises a superimposition:
- of Al₂O₃ in contact with the conductive layer (2), then
- of silicon oxide in contact with the Al₂O₃ of the passivation layer, such that the Al₂O₃ of the passivation layer is comprised between the conductive layer (2) and the silicon oxide of the passivation layer.

12. Method according to the preceding claim, **characterized in that** the passivation layer (8) has a breakdown field greater than or equal to 10⁶ V.m⁻¹.

13. Device, comprising:
- a substrate (3),
- a conductive layer (2) divided into at least one measurement area,
- at least two distinct electrodes (7) in contact with the conductive layer (2) **characterized in that**:
- the conductive layer (2) consists of a layer of a two-dimensional conductor having a doping level lower than 10¹¹ impurities per cm², and/or a shift of the Dirac point with respect to 0 volts smaller than 1 volt in absolute terms,
- it moreover comprises a passivation layer (8) having a breakdown field greater than or equal to 10⁶ V.m⁻¹ and covering the conductive layer (2) and at least part of each electrode (7), such that the conductive layer (2) and at least part of each electrode (7) are located between the substrate (3) and the passivation layer (8).

14. Device according to claim 13, **characterized in that** the conductive layer (2) is divided into several distinct measurement areas.

15. Device according to claim 14, **characterized in that** the electrodes (7) comprise a common source (71) in contact with all the measurement areas and one drain (72) per measurement area, each drain (72) being in contact with only a single measurement area.

16. Device according to any one of claims 13 to 15, **characterized in that** it moreover comprises a tank (9) arranged to receive an electrolyte (10) above the conductive layer (2),
preferably such that the electrolyte (10) is in contact with the passivation layer (8) or with the conductive layer (2).

17. Device according to claim 16, **characterized in that** it comprises means (11) for measuring and/or imposing a voltage (Vse) and/or an electric current between the electrolyte (10) and a source electrode (71).

18. Device according to any one of claims 13 to 17, **characterized in that** it comprises means (11) for measuring and/or imposing a voltage (V_{SG}) and/or an electric current between a gate (3) and a source electrode (71).

19. Device according to any one of claims 13 to 18, **characterized in that** it comprises means (12) for measuring and/or imposing a voltage (Vds) and/or an electric current (Ids) between a source electrode (71) and a drain electrode (72).

20. Device according to any one of claims 13 to 19, **characterized in that** it comprises a layer (13) of polymer and/or of nucleotides above the at least one measurement area, in contact with the conductive layer (2) or in contact with the passivation layer (8).

21. Method for using a device according to any one of claims 13 to 19 or obtained by means of a method according to any one of claims 1 to 12, comprising:
- a step of detecting the attachment of biological cells or atoms or molecules on or above the conductive layer (2) or the passivation layer (8), and/or
- a step of detecting an electrical activity of at least one biological cell, on or above the conductive layer or the passivation layer.

22. Method according to claim 21, **characterized in that** the attachment is an attachment of biological cells or atoms or molecules on a layer of polymer and/or of nucleotides attached to the conductive layer (2) or to the passivation layer (8).

23. Method according to claim 21 or 22, **characterized in that** the attachment is a hybridization of nucleotides on a layer (13) of polymer and of nucleotides located above the at least one measurement area, and in contact with the conductive layer (2) or in contact with the passivation layer (8).
